# EUROPEAN PATENT APPLICATION

(11) **EP 4 089 171 A1**
(43) Date of publication of application: **16.11.2022**
(21) Application number: 21382441.0
(22) Date of filing: 12.05.2021
(51) Int. Cl.: C12N 15/86, C12N 15/861, A61K 48/00

(54) **RECOMBINANT TERT-ENCODING VIRAL GENOMES AND VECTORS**

(71) Applicant: Fundación del Sector Público Estatal Centro Nacional de Investigaciones Oncológicas Carlos III (F.S.P. CNIO), 28029 Madrid (ES); Universitat Autònoma de Barcelona, 08193 Cerdanyola del Vallès (ES)
(72) Inventor: BLASCO, Maria, 28029 Madrid (ES); MARTINEZ, Paula, 28029 Madrid (ES); BOSCH TUBERT, Maria Fàtima, 08290 Cerdanyola del Valles (Barcelona) (ES); JIMENEZ CENZANO, Verónica, 08202 Sabadell (Barcelona) (ES); GARCIA MARTINEZ, Miquel, 08225 Terrassa (Barcelona) (ES); CASANA LORENTE, Estefanía, 08205 Sabadell (Barcelona) (ES)
(74) Representative: Hoffmann Eitle

(57) **Abstract**

Described herein are recombinant viral genomes comprising a nucleotide sequence encoding a telomerase reverse transcriptase (TERT) operably linked to a tissue-specific and/or organ-specific promoter. Aspects described herein may be used in the treatment of conditions associated with shortened telomere length, such as pulmonary fibrosis, myocardial infarction and conditions associated therewith.

## Description

### Field

Aspects and embodiments described herein relate to the field of medicine, particularly gene therapy.

### Background

Telomeres are specialized structures at the ends of chromosomes, which have a role in protecting the chromosome ends from DNA repair and degrading activities (Blackburn et al. Cell 2001; 106(6):661-673). Mammalian telomeres consist of TTAGGG repeats bound by a multi-protein complex known as shelterin. The presence of a minimum length of TTAGGG repeats and the integrity of the shelterin complex are necessary for telomere protection. When telomeres become critically short, they lose their protective function and a persistent DNA damage response at the telomeres is triggered which subsequently leads to a cellular senescence response.

Telomerase is a cellular reverse transcriptase (TERT, telomerase reverse transcriptase; also known as TP2; TRT; EST2; TCSI) capable of compensating telomere attrition through de novo addition of TTAGGG repeats onto the chromosome ends by using an associated ncRNA component as template (Terc, telomerase RNA component) (Greider & Blackburn. Cell 1985; 43:405-413). Telomerase is expressed in most adult stem cell compartments; however, in most somatic cells telomerase is silenced after birth leading to the gradual shortening of telomeres. This is evidenced by the fact that telomere shortening occurs with age in most human and mouse tissues (Blasco, Nat Chem Biol. 2007; 3:640-649).

Shortened telomeres have been associated with numerous diseases, such as dyskeratosis congenita, aplastic anaemia, myelodysplastic syndrome, Fanconi anaemia, pulmonary fibrosis and cardiovascular diseases (CVD). Given the severity of these diseases and the poor prognosis of the patients suffering from them, there is a need for improved therapies to treat diseases associated with shortened telomere length. Therapeutic intervention with telomerase, aimed at preventing telomere loss beyond a critically short length, represents a potential treatment for conditions associated with shortened telomeres. AAV-mediated TERT gene therapy such as using AAV9 vectors encoding telomerase under the control of the CMV promoter (AAV9-CMV-mTert), was previously shown to be effective in extending health span of mice (Bernardes de Jesus et al. EMBO Mol Med 2012; 4:691-704) and treating conditions associated with shortened telomere length such as aplastic anemia and pulmonary fibrosis (WO2016/20345, Beier et al. Blood 2012; 120(15):2990-3000, Povedano et al. eLife 2018; 7: e31299) and cardiovascular diseases (WO2016/020346, Bär et al. Nat Comm 2014; 5:5863), Chatterjee et al. Mol Ther 2021; 29(4): 1-16), demonstrated amelioration of doxorubicin toxicity in mice hearts and in human cardiomyocytes utilizing AAV9 vectors encoding telomerase under the control of the chicken troponin promoter. Existing AAV vectors are associated with drawbacks which affect their effectiveness in TERT gene therapy, such as low gene expression in target tissues, off-target gene expression in tissues other than the ones being treated and the occurrence of intermediate vector species containing truncated viral genomes. In view of the above, there is a need for the provision of improved recombinant viral genomes and vectors for gene therapy utilizing TERT.

### Summary

An aspect of the invention relates to a recombinant viral genome comprising a nucleotide sequence encoding a telomerase reverse transcriptase (TERT) operably linked to a tissue-specific and/or organ-specific promoter, wherein the total length of the viral genome is less than 4700 nucleotides. In some embodiments, the recombinant viral genome according to the invention is such that the tissue-specific and/or organ-specific promoter is a lung-specific promoter or a heart-specific promoter. In some embodiments, the recombinant viral genome according to the invention is an adeno-associated virus genome. In some embodiments, the promoter is selected from the group consisting of an SpB promoter, a CMVenh-MLC2V promoter, a troponin promoter and derivatives thereof. In some embodiments, the TERT encoding sequence is codon-optimized, preferably for expression in a human cell. In some embodiments, the recombinant viral genome according to the invention is such that:
a) the TERT encoding sequence comprises, essentially consists of, or consists of a nucleotide sequence encoding a polypeptide represented by an amino acid sequence having at least 60%, 70%, 80%, 90%, 95%, or 99% identity or similarity with any one of SEQ ID NOs: 4, 7, or 10, preferably with SEQ ID NO: 7 or 10, or;
b) the TERT encoding sequence comprises, essentially consists of, or consists of a nucleotide sequence having at least 60%, 70%, 80%, 90%, 95%, or 99% identity with any one of SEQ ID NOs: 1, 2, 3, 5, 6, 8, or 9 preferably with any one of SEQ ID NOs:3, 6, or 9, or;
c) the TERT encoding sequence comprises, essentially consists of, or consists of a nucleotide sequence the sequence of which differs from the sequence of a nucleotide sequence of a) or b) due to the degeneracy of the genetic code.

In some embodiments, the recombinant viral genome according to the invention is such that the promoter comprises, essentially consists of, or consists of a nucleotide sequence having at least 60%, 70%, 80%, 90%, 95%, or 99% identity with any one of the nucleotide sequences with SEQ ID NOs: 11, 12, 13, 15, or 16, preferably with SEQ ID NOs: 12, 15, or 16. In some embodiments, the recombinant viral genome further comprises a kozak consensus sequence operably linked to the nucleotide sequence encoding a TERT. In some embodiments, the recombinant viral genome further comprises a polyA sequence operably linked to the nucleotide sequence encoding a TERT.

Another aspect of the invention relates to an adeno-associated viral vector comprising a recombinant viral genome of the invention. In some embodiments, an adeno-associated viral vector of the invention is such that the viral vector is of serotype 1, 2, 3, 4, 5, 6, 7, 8, 9, rh10, rh8, Cb4, rh74, DJ, 2/5, 2/1, 1/2 or Anc80, preferably of serotype 6 or 9.

Another aspect of the invention relates to a pharmaceutical composition comprising a recombinant viral genome of the invention or an adeno-associated viral vector of the invention, optionally further comprising one or more pharmaceutically acceptable ingredients.

Another aspect of the invention relates to a recombinant viral genome of the invention, an adeno-associated viral vector of the invention, or a pharmaceutical composition of the invention, for use as a medicament. In some embodiments, a recombinant viral genome of the invention, an adeno-associated viral vector of the invention, or a pharmaceutical composition of the invention, is for use in the treatment and/or prevention of a condition associated with shortened telomere length. In some embodiments, a recombinant viral genome of the invention, an adeno-associated viral vector of the invention, or a pharmaceutical composition of the invention, is for use in the treatment and/or prevention of pulmonary fibrosis, myocardial infarction or conditions associated therewith.

### Description

The present invention provides recombinant viral genomes and vectors useful in gene therapy utilizing telomerase reverse transcriptase (TERT). Recombinant viral genomes and vectors described herein exhibit at least one, at least two, at least three, or all of the following benefits over known viral genomes and vectors:
- Increased TERT expression in target tissues
- Decreased TERT expression in off-target tissues
- Prevention of formation of intermediate species comprising truncated viral genomes associated with the utilization of viral vectors with low encapsidation capacity
- Increased therapeutic efficacy

As also demonstrated in the Examples section herein, a significant efficacy improvement over existing therapeutic strategies is expected from the application of the recombinant viral genomes and vectors of the invention in gene therapy utilizing TERT. Accordingly, the aspects and embodiments of the present invention as described herein solve at least some of the problems and needs as discussed herein.

### Recombinant viral genome

In a first aspect, there is provided a recombinant viral genome comprising a nucleotide sequence encoding a telomerase reverse transcriptase (TERT) operably linked to a tissue-specific and/or organ-specific promoter, wherein the total length of the viral genome is less than 4700 nucleotides.

A viral genome may consist of DNA or RNA molecules. Said molecules can be single or double stranded. Said molecules can be linear, circular, segmented (composed of multiple pieces of nucleic acids) or nonsegmented. Viral genome ends may comprise repeated sequences, chemical modifications and/or secondary structures, which may have regulatory functions such as assisting with viral genome replication in the respective host. In the context of the invention, any viral genome may be contemplated as long as it allows for expression of its comprised TERT-encoding nucleotide sequence upon introduction of said genome in a cell. In some embodiments, the viral genome originates from a lentivirus, an adenovirus, a herpes virus, a polyoma virus, a vaccinia virus, or an adeno-associated virus (AAV).

In preferred embodiments, the recombinant viral genome originates from an adeno-associated virus (AAV). Accordingly, in preferred embodiments, there is provided a recombinant AAV genome comprising a nucleotide sequence encoding a telomerase reverse transcriptase (TERT) operably linked to a tissue-specific and/or organ-specific promoter, wherein the total length of the viral genome is less than 4700 nucleotides. The term "adeno-associated viral genome" (AAV genome) refers to a genome of a virus that belongs to the genus *Dependoparvovirus* (also referred to as *Dependovirus*) of the family *Parvoviridae.* The naturally occurring (wild-type) AAV genome is approximately 4.7 kb in length. The genome includes inverted terminal repeats (ITRs) at both ends of the DNA strand.

A "recombinant", alternatively referred to as "chimeric" or "engineered", viral genome is a viral genome not normally found in nature (wild-type viral genome), such as a viral genome wherein native regulatory sequences and/or transcribed DNA regions have been removed, modified or otherwise juxtaposed in a manner not naturally occurring, and/or wherein non-native regulatory sequences and/or transcribed DNA regions have been incorporated.

The recombinant viral genome according to the invention optionally comprises inverted terminal repeats (ITRs) originating from an AAV genome. Said sequences may enhance the maintenance of the recombinant viral genome in a respective host. Inverted terminal repeats may be included at the 5' and the 3' flanks of the recombinant viral genome (5' ITR and 3' ITR, respectively). Suitable inverted terminal repeat sequences may be derived from any adeno-associated virus serotype, such as but not limited to AAV1, AAV2, AAV3, AAV4, AAV5, and others. Preferred ITRs are those of AAV2 which are represented by sequences comprising, consisting essentially of, or consisting of SEQ ID NO: 19 (5' ITR) and SEQ ID NO: 20 (3' ITR). The invention also preferably encompasses the use of a sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity with SEQ ID NO: 19 as 5' ITR and a sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity with SEQ ID NO: 20 as 3' ITR.

In some embodiments, a preferred recombinant viral genome is a recombinant adeno-associated viral genome (AAV genome). Said genome may or may not comprise its natively associated inverted terminal repeats (ITRs). Preferably, said genome comprises the ITRs of adeno-associated virus serotype 2 (AAV2), more preferably it comprises SEQ ID NO: 19 (5' ITR) and SEQ ID NO: 20 (3' ITR).

A recombinant viral genome according to the invention has a total length of less than 4700 nucleotides. The present inventors have surprisingly found that not exceeding the abovementioned length allows for the prevention of the formation of intermediate species comprising truncated viral genomes when viruses with low encapsidation capacity, such as but not limited to adeno-associated viruses, are utilized as expression vectors, resulting in improved therapeutic efficacy of said vectors. In some embodiments, the recombinant viral genome has a total length of less than 4680 nucleotides, less than 4660 nucleotides, less than 4640 nucleotides, less than 4620 nucleotides, less than 4600 nucleotides, less than 4580 nucleotides, less than 4560 nucleotides, less than 4540 nucleotides, less than 4520 nucleotides, or less than 4500 nucleotides, preferably less than 4680 nucleotides.

In some embodiments, the recombinant viral genome has a length of 4400-4700 nucleotides, 4420-4700 nucleotides, 4440-4700 nucleotides, 4460-4700 nucleotides, 4480-4700 nucleotides, 4500-4700 nucleotides, 4520-4700 nucleotides, 4540-4700 nucleotides, 4560-4700 nucleotides, 4580-4700 nucleotides, 4600-4700 nucleotides, 4620-4700 nucleotides, 4640-4700 nucleotides, 4660-4700 nucleotides, or 4680-4700 nucleotides, preferably it has a length of 4400-4700 nucleotides.

In some embodiments, the recombinant viral genome has a length of 4400-4700 +/- 10 nucleotides, 4420-4700 +/- 10 nucleotides, 4440-4700 +/- 10 nucleotides, 4460-4700 +/- 10 nucleotides, 4480-4700 +/- 10 nucleotides, 4500-4700 +/- 10 nucleotides, 4520-4700 +/- 10 nucleotides, 4540-4700 +/- 10 nucleotides, 4560-4700 +/- 10 nucleotides, 4580-4700 +/- 10 nucleotides, 4600-4700 +/- 10 nucleotides, 4620-4700 +/-10 nucleotides, 4640-4700 +/- 10 nucleotides, 4660-4700 +/- 10 nucleotides, or 4680-4700 +/- 10 nucleotides, preferably it has a length of 4400-4700 +/- 10 nucleotides.

A "telomerase reverse transcriptase" (TERT), alternatively known as TP2, TRT, EST2, or TCSI (EC 2.7.7.49) is a catalytic component of the telomerase holoenzyme complex, whose main activity is the elongation of telomeres by its acting as a reverse transcriptase that adds simple sequence repeats to chromosome ends by copying a template sequence within the ncRNA component of the enzyme (Terc, telomerase RNA component). TERT catalyzes the RNA-dependent extension of 3'-chromosomal termini with the 6-nucleotide telomeric repeat unit 5'-TTAGGG-3'. The skilled person understands that the term also encompasses modified versions of TERT, such as but not limited to TERT fragments, as long as said modified versions remain functional. In this context, functionality refers to the modified protein exhibiting at least 20%, at least 30%, at least 40%, at least 50%, at least 60%, at least 70%, at least 80%, at least 90%, or at least 95%, of the catalytic activity as compared to the unmodified protein. A modified version of TERT may also exhibit increased functionality as compared to the unmodified protein. In this context, increased functionality refers to the modified protein exhibiting at least 105%, at least 110%, at least 120%, at least 130%, at least 140%, at least 150%, at least 160%, at least 170%, at least 180%, at least 190%, or at least 200% of the catalytic activity as compared to the unmodified protein. Catalytic activity of TERT may be measured according to standard methods in the art as discussed elsewhere herein.

A nucleotide sequence encoding a TERT according to the invention preferably comprises, essentially consists of, or consists of, the coding sequence (CDS) of a TERT gene. "Coding sequence" or "coding region" refers to the part of a gene that codes for a protein. Said nucleotide sequence may be modified or non-modified compared to a naturally-occurring, alternatively referred to as "wild-type", sequence. Suitable non-limiting modifications may be selected from nucleotide insertions, deletions, mutations and/or substitutions. The skilled person understands that modifications resulting in the expression of modified versions of TERT as discussed above are also encompassed. The nucleotide sequence encoding a TERT may or may not comprise non-coding regions of a TERT gene, such as introns and/or 5' and/or 3' untranslated regions, preferably it does not comprise non-coding regions. Accordingly, in some embodiments the nucleotide sequence encoding a TERT comprises, essentially consists of, or consists of coding regions of a TERT gene. In some embodiments, the nucleotide sequence encoding a TERT consists of the coding sequence of a TERT gene. In some embodiments, a preferred nucleotide sequence encoding a TERT does not comprise the natively associated 3' UTR region of a TERT gene.

Modification of a nucleotide sequence may be performed using any recombinant DNA technique as known in the art, such as for example described in standard handbooks like Ausubel et al., Current Protocols in Molecular Biology, 3rd edition (2003), John Wiley & Sons Inc and in Sambrook and Green, Molecular Cloning. A Laboratory Manual, 4th Edition (2012), Cold Spring Harbor Laboratory Press; both of which are incorporated herein by reference in their entirety. Also see, Kunkel (1985) Proc. Natl. Acad. Sci. 82:488 (describing site directed mutagenesis) and Roberts et al. (1987) Nature 328:731-734 or Wells, J.A., et al. (1985) Gene 34: 315 (describing cassette mutagenesis).

A nucleotide sequence encoding a TERT that is comprised in a recombinant viral genome according to the invention may or may not correspond to a nucleotide sequence that is native to a cell wherein the recombinant viral genome comprising said gene is to be introduced in. Said nucleotide sequence may be derived from any TERT-encoding sequence. In some embodiments, the nucleotide sequence encoding a TERT is of mammalian origin. In some embodiments, the nucleotide sequence encoding a TERT is of murine, leporid, swine, equine, sheep, bovine, feline, canine, or human origin. In humans, two TERT isoforms exist which differ in length; a long isoform (NCBI CCDS ID: 3861.2) and a short isoform (NCBI CCDS ID:54831.1). In some embodiments, the nucleotide sequence encoding a TERT is of murine (such as mouse or rat) or human origin, preferably of human origin, more preferably it is the long or short isoform of human TERT or a modified version thereof.

Modification of a nucleotide sequence encoding TERT may comprise codon optimization. Accordingly, in some embodiments, the nucleotide sequence encoding a TERT is codon optimized, preferably for expression in a human cell or a mouse cell, preferably a human cell. A description of "codon optimization" is provided later herein in the section entitled "general information".

In some embodiments, a preferred nucleotide sequence encoding a TERT comprises, essentially consists of, or consists of a nucleotide sequence encoding a polypeptide represented by the amino acid sequence of SEQ ID NO: 4, or an amino acid sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity or similarity with SEQ ID NO: 4. SEQ ID NO: 4 represents an amino acid sequence of mouse TERT (Uniprot accession number: O70372-1).

In some embodiments, a preferred nucleotide sequence encoding a TERT comprises, essentially consists of, or consists of a nucleotide sequence encoding a polypeptide represented by the amino acid sequence of SEQ ID NOs: 7 or 10, or an amino acid sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity or similarity with SEQ ID NO: 7 or 10. SEQ ID NO: 7 represents an amino acid sequence of the long isoform of human TERT (Uniprot accession number: O14746-1). SEQ ID NO: 10 represents an amino acid sequence of the short isoform of human TERT (Uniprot accession number: O14746-3).

In some embodiments, a preferred nucleotide sequence encoding a TERT comprises, essentially consists of, or consists of a nucleotide sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity with any one of SEQ ID NOs: 1, 2, 3, 5, 6, 8, or 9, preferably with any one of SEQ ID NOs: 3, 6, or 9, more preferably with SEQ ID NO: 6 or 9.

Accordingly, in preferred embodiments the recombinant viral genome according to the invention comprises a nucleotide sequence encoding a telomerase reverse transcriptase (TERT), wherein:
a) the TERT encoding sequence comprises, essentially consists of, or consists of a nucleotide sequence encoding a polypeptide represented by the amino acid sequence of any one of SEQ ID NOs: 4, 7, or 10, preferably SEQ ID NO: 7 or 10, or an amino acid sequence having at least 60%, 70%, 80%, 90%, 95%, or 99% identity or similarity with any one of SEQ ID NOs: 4, 7, or 10, preferably with SEQ ID NO: 7 or 10, or;
b) the TERT encoding sequence comprises, essentially consists of, or consists of a nucleotide sequence having at least 60%, 70%, 80%, 90%, 95%, or 99% identity with any one of SEQ ID NOs: 1, 2, 3, 5, 6, 8, or 9, preferably with any one of SEQ ID NOs: 3, 6, or 9, more preferably with SEQ ID NO: 6 or 9, or;
c) the TERT encoding sequence comprises, essentially consists of, or consists of a nucleotide sequence the sequence of which differs from the sequence of a nucleotide sequence of a) or b) due to the degeneracy of the genetic code.

The nucleotide sequence encoding a telomerase reverse transcriptase (TERT) according to the invention is operably linked to a tissue-specific and/or an organ-specific promoter. A description of "operably linked" is provided later herein in the section entitled "general information". Throughout this disclosure, the terms "promoter" and "promoter sequence" are used interchangeably and may be replaced by "transcription regulatory sequence" or "regulatory sequence". Definitions of the terms are provided in the "general information" section.

A "tissue-specific" or "organ-specific" promoter is a promoter that is active in a specific type of tissue or organ, respectively. Said promoter may be constitutive or inducible. Definitions of the terms are provided in the "general information" section. The term also encompasses promoters that are active in specific cells of said tissues or organs (cell-specific promoters). In this context, the term "promoter activity" refers to the capability of a promoter to initiate transcription of a coding nucleotide sequence operably linked to said promoter. Tissue-specific and organ-specific promoters regulate expression of one or more genes primarily in one tissue or organ and/or in specific cells of a tissue or organ. In some embodiments, they may still allow detectable level ("leaky") expression in other tissues or organs. Leaky expression in other tissues or organs means at least one-fold, at least two-fold, at least three-fold, at least four-fold or at least five-fold lower, but still detectable expression as compared to the tissue-specific or organ-specific expression, as evaluated on the level of the mRNA or the protein by standard assays known to a person of skill in the art (e.g. qPCR, Western blot analysis, ELISA).

Assessment of the tissue-specific and/or organ-specific nature of a promoter can be performed by standard molecular toolbox techniques, such as, for example, described in Sambrook and Green (supra). As a non-limiting example, any expression vector comprising any of the recombinant viral genomes as described herein, wherein the nucleotide sequence encoding a TERT has been replaced by a nucleotide sequence encoding a GFP, can be produced. Cells, tissues and organs transduced as described herein can then be assessed for expression based on RNA or protein levels or immunostaining and/or microscopy methods (e.g. fluorescence microscopy, light microscopy, bright-field microscopy, and the like) according to standard protocols, for example as described in the Examples section of this disclosure.

A promoter sequence that is operably linked to a nucleotide sequence encoding a TERT according to the invention may or may not correspond to a promoter sequence that is native to a cell wherein the recombinant viral genome comprising said promoter is to be introduced in. In some embodiments, the promoter sequence is of mammalian origin. In some embodiments, the promoter sequence is of murine, leporid, swine, equine, sheep, bovine, feline or canine origin. In some embodiments, the promoter sequence is of murine (such as mouse or rat) or human origin, preferably of human origin.

As demonstrated in the Examples, the present inventors have surprisingly found that the tissue- and organ-specific promoters described herein allow for increased gene expression in target tissues and decreased gene expression in off-target tissues.

In some embodiments, the tissue-specific and/or organ-specific promoter according to the invention is a lung-specific promoter or a heart-specific promoter, preferably it is a lung-specific promoter.

A "lung-specific promoter" is a promoter that is capable of initiating transcription in the lung and/or a lung tissue and/or a lung cell. Said promoter may or may not still allow leaky expression in other organs and parts of the body. "Lung-specific" also encompasses promoters that drive the preferential or predominant expression of a nucleotide sequence in the lung and/or a lung tissue and/or a lung cell as compared to other organs, tissues or cells as described later herein. Transcription in the lung can be detected in relevant areas, such as the trachea, bronchi, pleura, diaphragm, bronchioles, alveoli or epithelium. Promoters that are capable of initiating transcription in cells of the lung epithelium are advantageous. Promoters that are capable of initiating transcription in alveolar type II epithelial cells (ATII cells) are particularly advantageous. Accordingly, in some embodiments, the lung-specific promoter is an alveolar type II epithelial cell specific (ATII-cell specific) promoter. In some embodiments, a preferred lung-specific promoter is selected from the group consisting of a surfactant protein A (SpA) promoter, a surfactant protein B (SpB) promoter, and derivatives thereof, preferably it is a SpB promoter or a derivative thereof.

A "heart-specific promoter", alternatively known as "cardiac-specific promoter', is a promoter that is capable of initiating transcription in the heart and/or a heart tissue and/or a heart cell. Said promoter may or may not still allow leaky expression in other organs and parts of the body. "Heart-specific" also encompasses promoters that drive the preferential or predominant expression of a nucleotide sequence in the heart and/or a heart tissue and/or a heart cell as compared to other organs, tissues or cells as described later herein. Transcription in the heart can be detected in relevant areas, such as the tricuspid valve, pulmonary valve, mitral valve, aortic valve, left atrium, right atrium, left ventricle, right ventricle, epithelium or myocardium.

Promoters that are capable of initiating transcription in cells of the myocardium are advantageous. Promoters that are capable of initiating transcription in cardiac myocytes are particularly advantageous. Accordingly, in some embodiments, the heart-specific promoter is a myocardium-specific promoter. In some embodiments, the heart-specific promoter is a cardiac myocyte-specific promoter. In some embodiments, a preferred heart-specific promoter is selected from the group consisting of the myosin light chain 2v (MLC2v), troponin (TNT) promoters and derivatives thereof, preferably it is the troponin (TNT) promoter or a derivative thereof. In some embodiments, a preferred myocardium-specific promoter or a cardiac myocyte-specific promoter is selected from the group consisting of the myosin light chain 2v (MLC2v), troponin (TNT) promoters and derivatives thereof, preferably it is the troponin (TNT) promoter or a derivative thereof.

In the context of the invention, tissue-specific and/or organ-specific promoters may be promoters that are capable of driving the preferential or predominant (at least 10% higher, at least 20% higher, at least 30% higher, at least 40% higher, at least 50% higher, at least 60% higher, at least 70% higher, at least 80% higher, at least 90% higher, at least 100% higher, at least 150% higher, at least 200% higher or more) expression of a nucleotide sequence in the specific tissues and/or organs as compared to other tissues or organs. Other organs or tissues may be the liver, CNS, brain, adipose tissue (white and/or brown), skeletal muscle, heart, kidney, colon, hematopoietic tissue, lung, ovary, spleen, stomach, pancreas, testis, epididymis, intestine, and others. Preferably, other organs or tissues are the liver and adipose tissue (white and/or brown).

A lung-specific promoter may preferably be a promoter that is capable of driving the preferential or predominant expression of a nucleotide sequence in the lungs as compared to one or more tissues and/or organs selected from the brain, adipose tissue (white and/or brown), heart and/or liver, preferably brain, brown adipose tissue and/or heart. A heart-specific promoter may preferably be a promoter that is capable of driving the preferential or predominant expression of a nucleotide sequence in the heart as compared to one or more tissues and/or organs selected from the adipose tissue (white and/or brown), liver, intestine, testis and/or skeletal muscle. Expression may be assessed as described in the "general information" section.

The nucleotide sequences of the promoters according to the invention may be modified as compared to the corresponding naturally-occurring sequences. Such modified promoters may herein be alternatively referred to as "derivatives" of their naturally-occurring (wild-type) versions. Suitable non-limiting modifications may be selected from nucleotide insertions, deletions, mutations and/or substitutions. Suitable modifications also encompass promoter sequence fusions with other nucleotide sequences, such as but not limited to enhancer or other promoter sequences, as discussed elsewhere herein. Particularly advantageous are modifications that result in a promoter sequence that is minimized in length ("shortened promoter" or "promoter fragment") as compared to the corresponding naturally-occurring promoter sequence. A shortened promoter may preferably comprise at least 5%, at least 10%, 12%, 14%, 16%, 18%, 20%, 22%, 24%, 26%, 28%, 30%, 32%, 34%, 36%, 38% or 40% of the sequence length of the corresponding naturally-occurring promoter sequence.

In some embodiments, a tissue-specific and/or organ-specific promoter as described herein may have a length of less than 1000 nucleotides, less than 900 nucleotides, less than 800 nucleotides, less than 700 nucleotides or less than 650 nucleotides.

In some embodiments, a tissue-specific and/or organ-specific promoter as described herein may have a length of 100-1000 nucleotides, 200-900 nucleotides, 300-800 nucleotides, 400-700 nucleotides or 350-650 nucleotides.

In preferred embodiments, the lung-specific promoter is a shortened version of the SpB promoter, preferably of the human SpB promoter. In some embodiments, a preferred lung-specific promoter comprises at least 10%, 12%, 14%, 16%, 18%, 20%, 22%, 24%, 26%, 28%, 30%, 32%, 34%, 36%, 38% or 40%, preferably at least 38% or 40% of the sequence length of SEQ ID NO: 11, more preferably it comprises SEQ ID NO: 12, or a sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity therewith.

In some embodiments, a preferred heart-specific promoter is a shortened version of the MLC2v promoter, preferably of the human MLC2v promoter, more preferably it is a 296-nucleotide fragment of the human MLC2v promoter. In some embodiments, a 296-nucleotide fragment of the human MLC2v promoter has the sequence of SEQ ID NO: 13 or a sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity therewith.

In some embodiments, a heart-specific promoter is not the 250-nucleotide fragment of the rat MLC2v promoter as described in Henderson et al. J Biol Chem 264;30:18142-8, (1989) (incorporated herein by reference in its entirety) and/or a heart-specific promoter is not the 281-nucleotide fragment (corresponding to nucleotides -264 to +17 of Genebank Accession No: U26708) of the rat MLC2v promoter as described in Ian Phillips et al. Hypertension 39:651-655, (2002) (incorporated herein by reference in its entirety).

In some embodiments, a preferred heart-specific promoter is a shortened version of the troponin promoter, preferably of the human troponin promoter, more preferably it is the 544-bp fragment of the human troponin promoter described in Werfel et al. Cardiovasc Res 104;1:15-23, (2014) (SEQ ID NO: 16), incorporated herein by reference in its entirety, or a sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity therewith.

A derivative of a promoter according to the invention preferably exhibits at least 10%, at least 20%, at least 30%, at least 40%, at least 50%, at least 60%, at least 70%, at least 80%, at least 90%, or at least 95% of the transcription initiation capability of the corresponding naturally-occurring sequences. Sequence modification methods and methods of assessing transcription initiation capability ("promoter strength") are discussed elsewhere herein.

A promoter or derivative thereof as discussed herein may be present in single or multiple copies in the recombinant viral genome.

A recombinant viral genome according to the invention may further comprise additional nucleotide sequences that are operably linked to the nucleotide sequence encoding TERT, such as, but not limited to, signal sequences, nuclear localization signals, expression enhancers, polyadenylation signals, kozak sequences and the like. Said sequences may or may not correspond to sequences that are native to a cell wherein the recombinant viral genome comprising said promoter is to be introduced in.

An "enhancer" is a sequence that can stimulate the transcription of the sequence it is operably linked to. Generally, DNA sequences that are operably linked are contiguous, and, in the case of a signal sequence, both contiguous and sharing a reading frame. However, enhancers need not be contiguous with a coding sequence whose transcription they control. Linking is accomplished by ligation at convenient restriction sites or at adapters or linkers inserted in lieu thereof, or by gene synthesis. Enhancers may be used as single sequences or may be comprised in a fusion nucleotide sequence with other enhancers and/or any of the tissue-specific and/or organ-specific promoters described herein. Enhancer-promoter fusions may be particularly advantageous in enhancing transcription-driving capability when shortened promoter derivatives, as compared to naturally-occurring promoters, are utilized to drive expression of a nucleotide sequence encoding a TERT.

In some embodiments, the recombinant viral genome of the invention comprises a CMV enhancer (SEQ ID NO: 14). In some embodiments, the recombinant viral genome of the invention comprises a fusion between a CMV enhancer and a shortened version of the MLC2v promoter, henceforth alternatively referred to as a CMVenh-MLC2v promoter. Preferably, said fusion is between a CMV enhancer and a 296-nucleotide fragment of the human MLC2v promoter as discussed elsewhere herein (SEQ ID NO: 15).

Accordingly, in some embodiments a recombinant viral genome according to the invention comprises a promoter which is selected from the group consisting of an SpB, a CMVenh-MLC2v, a troponin promoter and derivatives thereof, preferably said promoter is an SpB promoter or a derivative thereof.

In some embodiments, a heart-specific promoter, more particularly a troponin promoter, is not a chicken troponin promoter. In some embodiments, a heart-specific promoter, more particularly a troponin promoter, is not the 411 -nucleotide fragment of the chicken troponin promoter as described in Prasad et al. Gene Ther 18(1):43-52, (2011), incorporated herein by reference in its entirety. In some embodiments, a heart-specific promoter, more particularly a troponin promoter, is not the chicken troponin promoter as described in Chatterjee, S., et al. Mol Ther 29(4): 1-16, (2021), incorporated herein by reference in its entirety.

In some embodiments, a lung-specific promoter comprises, essentially consists of, or consists of a nucleotide sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity with SEQ ID: NO 11 or 12, preferably with SEQ ID NO: 12.

In some embodiments, a heart-specific promoter comprises, essentially consists of, or consists of a nucleotide sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity with any one of SEQ ID NOs: 13, 15, or 16, preferably with SEQ ID NO: 15 or 16, more preferably with SEQ ID NO: 16.

Accordingly, in preferred embodiments a recombinant viral genome according to the invention comprises a promoter that comprises, essentially consists of, or consists of a nucleotide sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity with any one of the nucleotide sequences with SEQ ID NOs: 11, 12, 13, 15, or 16, preferably with SEQ ID NOs: 12, 15, or 16, more preferably with SEQ ID NO: 12 or SEQ ID NO: 16, most preferably with SEQ ID NO: 12.

A kozak sequence, alternatively referred to herein as a kozak consensus sequence, is a nucleic acid motif that functions as the protein translation initiation site and can enhance the expression of nucleotide sequences it is operably linked to. In some embodiments, a recombinant viral genome according to the invention comprises a kozak consensus sequence operably linked to the nucleotide sequence encoding a TERT. Preferably, said kozak consensus sequence is of human origin. In some embodiments, a preferred kozak consensus sequence comprises, essentially consists of, or consists of a nucleotide sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity with SEQ ID NO: 17.

A polyadenylation sequence, alternatively referred to herein as a "polyA sequence" or "polyA tail", is a nucleotide sequence consisting of a stretch of multiple adenosine monophosphates that can enhance the expression of nucleotide sequences it is operably linked to by enhancing nuclear export, translation and/or stability of mRNA sequences. In some embodiments, a recombinant viral genome according to the invention comprises a polyA sequence operably linked to the nucleotide sequence encoding a TERT. Preferably, said polyA sequence is selected from the SV40 polyA sequence, the rabbit β-globin polyA sequence, or the bovine growth hormone polyA sequence, more preferably it is the bovine growth hormone polyA sequence. In some embodiments, a preferred polyA sequence comprises, essentially consists of, or consists of a nucleotide sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity with SEQ ID NO: 18.

In some embodiments, a preferred recombinant viral genome is a recombinant AAV genome comprising a nucleotide sequence encoding a telomerase reverse transcriptase (TERT) operably linked to a lung-specific promoter, preferably to a SpB promoter or a derivative thereof, more preferably to a promoter comprising, essentially consisting of, or consisting of a nucleotide sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity with SEQ ID: NO 11 or 12, preferably with SEQ ID NO: 12, wherein the total length of the viral genome is less than 4700 nucleotides. Optionally, the gene construct further includes 5' and 3' flanks of inverted terminal repeats (ITRs) derived from the genome of an AAV, preferably from AAV2. In some embodiments, such a recombinant viral genome comprises, essentially consists of, or consists of, the nucleotide sequence of SEQ ID NO: 21, or a sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity therewith.

In some embodiments, a preferred recombinant viral genome is a recombinant AAV genome comprising a nucleotide sequence encoding a telomerase reverse transcriptase (TERT) operably linked to a heart-specific promoter, preferably to a CMVenh-MLC2v promoter or derivative thereof, more preferably to a promoter comprising, essentially consisting of, or consisting of a nucleotide sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity with SEQ ID: NO 15, wherein the total length of the viral genome is less than 4700 nucleotides. Optionally, the gene construct further includes 5' and 3' flanks of inverted terminal repeats (ITRs) derived from the genome of an AAV, preferably from AAV2. In some embodiments, such a recombinant viral genome comprises, essentially consists of, or consists of the nucleotide sequence of SEQ ID NO: 22, or a sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity therewith.

In some embodiments, a preferred recombinant viral genome is a recombinant AAV genome comprising a nucleotide sequence encoding a telomerase reverse transcriptase (TERT) operably linked to a heart-specific promoter, preferably to a troponin promoter or a derivative thereof, more preferably to a promoter comprising, essentially consisting of, or consisting of a nucleotide sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity with SEQ ID: NO 16, wherein the total length of the viral genome is less than 4700 nucleotides. Optionally, the gene construct further includes 5' and 3' flanks of inverted terminal repeats (ITRs) derived from the genome of an AAV, preferably from AAV2. In some embodiments, such a recombinant viral genome comprises, essentially consists of, or consists of the nucleotide sequence of SEQ ID NO: 23, or a sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity therewith.

The tissue-specific and/or organ-specific promoters described herein are useful for tissue and/or organ specific gene expression. Accordingly, in a further aspect there is provided a tissue-specific and/or organ-specific promoter as described herein. In some embodiments, the tissue-specific and/or organ-specific promoter is a lung-specific promoter, preferably an SpB promoter or a derivative thereof as described herein, more preferably a shortened version of the SpB promoter as described herein, even more preferably of the human SpB promoter as described herein. In some embodiments, a preferred lung-specific promoter comprises at least 5%, 10%, 12%, 14%, 16%, 18%, 20%, 22%, 24%, 26%, 28%, 30%, 32%, 34%, 36%, 38% or 40%, preferably at least 38% or 40% of the sequence length of SEQ ID NO: 11, more preferably it comprises SEQ ID NO: 12. In some embodiments, the tissue-specific and/or organ-specific promoter is a heart-specific promoter, preferably an MLC2v promoter or a derivative thereof as described herein, more preferably a shortened version of the MLC2v promoter as described herein, even more preferably of the human MLC2v promoter as described herein. In some embodiments, a heart-specific promoter comprises SEQ ID NO: 13. In some embodiments, a heart-specific promoter comprises SEQ ID NO: 15.

For any sequence described herein, in some embodiments, the level of sequence identity or similarity as used herein is preferably 70%. Another preferred level of sequence identity or similarity is 80%. Another preferred level of sequence identity or similarity is 90%. Another preferred level of sequence identity or similarity is 95%. Another preferred level of sequence identity or similarity is 99%.

### Expression vector

A recombinant viral genome as described herein can be placed in expression vectors. Thus, in a further aspect there is provided an expression vector comprising a recombinant viral genome as described herein. A description of "expression vector" is provided in the section entitled "general information". The skilled person understands that the term "expression vector" includes non-viral and viral vectors. Suitable expression vectors may be selected from any genetic element which can facilitate transfer of genes or nucleic acids between cells, such as, but not limited to, a plasmid, phage, transposon, cosmid, chromosome, artificial chromosome, virus, virion, and the like. A suitable expression vector may also be a chemical vector, such as a lipid complex or naked DNA. "Naked DNA" or "naked nucleic acid" refers to a nucleic acid molecule that is not contained within a viral particle, bacterial cell, or other encapsulating means that facilitates delivery of nucleic acid into the cytoplasm of the target cell. Optionally, a naked nucleic acid can be associated with standard means used in the art for facilitating its delivery of the nucleic acid to the target cell, for example to facilitate the transport of the nucleic acid through the alimentary canal, to protect the nucleic acid from stomach acid and/or nucleases, and/or serve to penetrate intestinal mucus.

In preferred embodiments, the expression vector is a viral expression vector. A description of "viral expression vector" has been provided under the section entitled "general information". A viral vector may be a viral vector selected from the group consisting of adenoviral vectors, adeno-associated viral vectors, retroviral vectors and lentiviral vectors.
In preferred embodiments, the expression vector comprising a recombinant viral genome, preferably a recombinant AAV genome, as described herein is an adeno-associated viral vector. An adeno-associated viral vector may alternatively be referred to herein as an adeno-associated vector or adeno-associated virus derived vector or AAV vector. A description of "adeno-associated viral vector" has been provided under the section entitled "general information". Said vectors typically have a nucleotide sequence encapsidation capacity of approximately 4700 nucleotides (4.7 kb). As demonstrated in the Examples, the present inventors have surprisingly found that the recombinant viral genome of the invention exhibits significant unexpected advantages when AAV vectors are utilized as expression vectors, as the formation of intermediate species containing truncated viral genomes is prevented and the therapeutic efficacy of said vectors is increased. A preferred AAV vector may be selected from the list consisting of AAV of serotype 1 (AAV1), AAV of serotype 2 (AAV2), AAV of serotype 3 (AAV3), AAV of serotype 4 (AAV4), AAV of serotype 5 (AAV5), AAV of serotype 6 (AAV6), AAV of serotype 7 (AAV7), AAV of serotype 8 (AAV8), AAV of serotype 9 (AAV9), AAV of serotype rh10 (AAVrh10), AAV of serotype rh8 (AAVrh8), AAV of serotype Cb4 (AAVCb4), AAV of serotype rh74 (AAVrh74), AAV of serotype DJ (AAVDJ), AAV of serotype 2/5 (AAV2/5), AAV of serotype 2/1 (AAV2/1), AAV of serotype 1/2 (AAV1/2) and AAV of serotype Anc80 (AAVAnc80), preferably it is selected from the list consisting of AAV of serotype 6, 8, or 9 (AAV6, AAV8, or AAV9), more preferably it is selected from AAV of serotype 6 (AAV6) or 9 (AAV9), even more preferably it is of serotype 9 (AAV9)..

In some embodiments, a preferred expression vector is an AAV6 or AAV9, preferably an AAV9, and comprises a recombinant AAV genome comprising a nucleotide sequence encoding a telomerase reverse transcriptase (TERT) operably linked to a lung-specific promoter, wherein the total length of the viral genome is less than 4700 nucleotides as described earlier herein. Optionally, the recombinant AAV genome further includes 5' and 3' flanks of inverted terminal repeats (ITRs) derived from the genome of an AAV, preferably from AAV2. Said expression vector preferably comprises a nucleotide sequence encoding a TERT that comprises, essentially consists of, or consists of a nucleotide sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity with any one of SEQ ID NOs:1, 2, 3, 5, 6, 8, or 9,, preferably with any one of SEQ ID NOs: 3, 6, or 9, more preferably with SEQ ID NO: 46 or 9..

In some embodiments, a preferred expression vector is an AAV6 or AAV9, preferably an AAV9, and comprises a recombinant AAV viral genome comprising a nucleotide sequence encoding a telomerase reverse transcriptase (TERT) operably linked to a SpB promoter or derivative thereof, wherein the total length of the viral genome is less than 4700 nucleotides as described earlier herein. Preferably, the promoter sequence comprises, essentially consists of, or consists of a nucleotide sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity with SEQ ID: NO: 11 or 12, preferably with SEQ ID NO: 12. Optionally, the recombinant AAV viral genome further includes 5' and 3' flanks of inverted terminal repeats (ITRs) derived from the genome of an AAV, preferably from AAV2. Said expression vector preferably comprises a nucleotide sequence encoding a TERT that comprises, essentially consists of, or consists of a nucleotide sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity with any one of SEQ ID NOs: 1, 2, 3, 5, 6, 8, or 9, preferably with any one of SEQ ID NOs: 3, 6, or 9, more preferably with SEQ ID NO: 6 or 9.

In some embodiments, a preferred expression vector is an AAV6 or AAV9, preferably an AAV9, and comprises a recombinant AAV viral genome comprising a nucleotide sequence encoding a telomerase reverse transcriptase (TERT) operably linked to a heart-specific promoter, wherein the total length of the viral genome is less than 4700 nucleotides as described earlier herein. Optionally, the recombinant AAV viral genome further includes 5' and 3' flanks of inverted terminal repeats (ITRs) derived from the genome of an AAV, preferably from AAV2. Said expression vector preferably comprises a nucleotide sequence encoding a TERT that comprises, essentially consists of, or consists of a nucleotide sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity with any one of SEQ ID NOs: 1, 2, 3, 5, 6, 8, or 9, preferably with any one of SEQ ID NOs: 3, 6, or 9, more preferably with SEQ ID NO: 6 or 9.

In some embodiments, a preferred expression vector is an AAV6 or AAV9, preferably an AAV9, and comprises a recombinant AAV viral genome comprising a nucleotide sequence encoding a telomerase reverse transcriptase (TERT) operably linked to a MLC2v promoter or derivative thereof, wherein the total length of the viral genome is less than 4700 nucleotides as described earlier herein. Preferably, the promoter sequence comprises, essentially consists of, or consists of a nucleotide sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity with SEQ ID NO: 13 or 15, preferably with SEQ ID NO: 15. Optionally, the recombinant AAV viral genome further includes 5' and 3' flanks of inverted terminal repeats (ITRs) derived from the genome of an AAV, preferably from AAV2. Said expression vector preferably comprises a nucleotide sequence encoding a TERT that comprises, essentially consists of, or consists of a nucleotide sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity with any one of SEQ ID NOs: 1, 2, 3, 5, 6, 8, or 9, preferably with any one of SEQ ID NOs: 3, 6, or 9, more preferably SEQ ID NO: 6 or 9..

In some embodiments, a preferred expression vector is an AAV6 or AAV9, preferably an AAV9, and comprises a recombinant AAV viral genome comprising a nucleotide sequence encoding a telomerase reverse transcriptase (TERT) operably linked to a troponin promoter or derivative thereof, wherein the total length of the viral genome is less than 4700 nucleotides as described earlier herein. Preferably, the promoter sequence comprises, essentially consists of, or consists of a nucleotide sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity with SEQ ID NO: 16. Optionally, the recombinant AAV viral genome further includes 5' and 3' flanks of inverted terminal repeats (ITRs) derived from the genome of an AAV, preferably from AAV2. Said expression vector preferably comprises a nucleotide sequence encoding a TERT that comprises, essentially consists of, or consists of a nucleotide sequence having at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% identity with any one of SEQ ID NOs: 1, 2, 3, 5, 6, 8, or 9, preferably with any one of SEQ ID NOs: 3, 6, or 9, more preferably with SEQ ID NO: 6 or 9.

The production of a recombinant AAV vector (also referred to as "rAAV") for vectorizing nucleotide sequences has been described previously, see Ayuso E, et al., Curr. Gene Ther. 2010; 10:423-436, Okada T, et al., Hum. Gene Ther. 2009; 20:1013-1021, Zhang H, et al., Hum. Gene Ther. 2009; 20:922-929, and Virag T, et al., Hum. Gene Ther. 2009; 20:807-817; all of which incorporated herein by reference in their entirety. These protocols can be used or adapted to generate an AAV vector according to the invention. Thus, in another aspect there is provided a method for producing an adeno-associated viral vector as described herein.
In short, the methods generally involve (a) the introduction of the recombinant AAV genome comprising the nucleotide sequence to be expressed into a cell, (b) the presence or introduction of an AAV helper construct in the cell, wherein the helper construct comprises the viral functions missing from the recombinant AAV genome and, optionally, (c) the introduction of a helper virus and/or helper virus plasmid into the host cell. All components for AAV vector replication and packaging need to be present, to achieve replication and packaging of the genome into AAV vectors. These typically include AAV cap proteins, AAV rep proteins and, optionally, viral proteins upon which AAV is dependent for replication. Rep and cap regions are well known in the art, see e.g. Chiorini et al. (1999, J. of Virology, Vol 73(2): 1309-1319, incorporated herein by reference in its entirety) or US 5,139,941 (incorporated herein by reference in its entirety). The AAV cap and rep proteins may derive from the same AAV serotype or they can derive from a combination of different serotypes, preferably they derive from an AAV6 or AAV9 serotype, more preferably from an AAV9 serotype. The viral proteins upon which AAV is dependent for replication may derive from any virus, such as a herpes simplex viruses (such as HSV types 1 and 2), a vaccinia virus, an adeno-associated virus or an adenovirus, preferably from an adenovirus.

In some embodiments, the producer cell line is transfected transiently with a recombinant AAV genome according to the invention (comprising the TERT expression cassette flanked by ITRs) and with construct(s) that encode(s) rep and cap proteins and provide(s) helper functions (helper construct(s)). In some embodiments, the cell line supplies stably the helper functions and is transfected transiently with the recombinant AAV genome according to the invention (comprising the TERT expression cassette flanked by ITRs) and with construct(s) that encode(s) rep and cap proteins. In some embodiments, the cell line supplies stably the rep and cap proteins and the helper functions and is transiently transfected with the recombinant AAV genome according to the invention. In another embodiment, the cell line supplies stably the rep and cap proteins and is transfected transiently with the recombinant AAV viral genome according to the invention and a polynucleotide encoding the helper functions. In some embodiments, the cell line supplies stably the recombinant AAV genome according to the invention, the rep and cap proteins and the helper functions. Methods of making and using these and other AAV production systems have been described in the art. See Muzyczka N, et al., US 5,139,941, Zhou X, et al., US 5,741,683, Samulski R, et al., US 6,057,152, Samulski R, et al., US 6,204,059, Samulski R, et al., US 6,268,213, Rabinowitz J, et al., US 6,491,907, Zolotukhin S, et al., US 6,660,514, Shenk T, et al., US 6,951,753, Snyder R, et al., US 7,094,604, Rabinowitz J, et al., US 7,172,893, Monahan P, et al., US 7,201,898, Samulski R, et al., US 7,229,823, and Ferrari F, et al., US 7,439,065, all of which are incorporated herein by reference in their entirety.

The recombinant AAV (rAAV) genome present in a rAAV vector typically comprises at least the nucleotide sequences of the inverted terminal repeat regions (ITRs) of one of the AAV serotypes (preferably the ones of serotype AAV2 as disclosed herein), or nucleotide sequences substantially identical thereto or nucleotide sequences having at least 60%, 70%, 80%, 90%, 95% or 99% identity thereto, and a nucleotide sequence encoding a TERT operably linked to a tissue-specific and/or organ-specific promoter as described herein, inserted between the two ITRs. A vector genome generally requires the use of flanking 5' and a 3' ITR sequences to allow for efficient packaging of the vector genome into the rAAV capsid. Said ITR sequences do not necessarily have to be derived from the same AAV serotype as the rAAV vector.

The complete genome of several AAV serotypes and corresponding ITRs has been sequenced (Chiorini et al. 1999, J. of Virology Vol. 73, No.2, p1309-1319, incorporated herein by reference in its entirety). They can be either cloned or made by chemical synthesis as known in the art, using for example an oligonucleotide synthesizer as supplied e.g. by Applied Biosystems Inc. (Fosters, CA, USA) or by standard molecular biology techniques. The ITRs can be cloned from an AAV genome or excised from a vector comprising the AAV ITRs. The ITR nucleotide sequences can be either ligated at either end of a nucleotide sequence encoding a TERT operably linked to a tissue-specific and/or organ-specific promoter as described herein using standard molecular biology techniques, or alternatively the AAV sequence between the ITRs can be replaced with the desired nucleotide sequence.

Preferably, the rAAV genome as present in a rAAV vector does not comprise any nucleotide sequences encoding viral proteins, such as the rep (replication) or cap (capsid) genes of AAV. This rAAV genome may further comprise a marker or reporter gene, such as a gene for example encoding an antibiotic resistance gene, a fluorescent protein (e.g. GFP) or a gene encoding a chemically, enzymatically or otherwise detectable and/or selectable product (e.g. lacZ, aph, etc.) known in the art.

The introduction into a producer cell can be carried out using standard virological techniques, such as transformation, transduction and transfection. Most vectors do not replicate in the producer cells infected with the vector. Examples of workable combinations of cell lines and expression vectors are described in Sambrook and Green (supra), and in Metzger et al (1988) Nature 334: 31-36 (incorporated herein by reference in its entirety). For example, suitable expression vectors can be expressed in, yeast, e.g. S. *cerevisiae,* e.g., insect cells, e.g., Sf9 cells, mammalian cells, e.g., CHO cells and bacterial cells, e.g., *E. coli.* A cell may thus be a prokaryotic or eukaryotic producer cell. A cell may be a cell that is suitable for culture in liquid or on solid media. Finally, the producer cells are cultured under standard conditions known in the art to produce the assembled AAV vectors which are then purified using standard techniques such as polyethylene glycol precipitation or CsCI gradients (Xiao et al. 1996, J. Virol. 70: 8098-8108, incorporated herein by reference in its entirety). Residual helper virus activity can be inactivated using known methods, such as for example heat inactivation.

The recombinant viral genomes and expression vectors as described herein may then be introduced into a host cell using standard molecular techniques, as discussed in standard handbooks such as Current Protocols in Molecular Biology (Ausubel et al., supra) and Sambrook and Green (supra). In the case of viral vectors, transduction is preferably used. The transduced host cell may or may not comprise the protein shell of the viral vectors. "Host cell" or "target cell", alternatively referred to as "cell" or "engineered cell", refers to the cell into which the DNA delivery takes place, such as the lung or heart cells of a mammalian subject as described elsewhere herein. AAV vectors in particular are able to transduce both dividing and non-dividing cells.
Accordingly, the invention further provides a host cell transduced with any of the recombinant viral genomes or expression vectors described herein. In some embodiments, a host cell transduced with any of the recombinant viral genomes or expression vectors described herein is lung cell, such as a lung cell of a vertebrate, preferably a lung cell of a mammal, more preferably an alveolar type II epithelial cell (ATII cell), of a mammal. In some embodiments, a host cell transduced with any of the recombinant viral genomes or expression vectors described herein is a lung cell, preferably an alveolar type II epithelial cell (ATII cell), of a rat, mouse, dog or a human, preferably of a mouse or a human, more preferably a human. In some embodiments, a host cell transduced with any of the recombinant viral genomes or expression vectors described herein is heart (cardiac) cell, such as a heart cell of a vertebrate, preferably a heart cell of a mammal, more preferably a myocardial cell of a mammal. In some embodiments, a host cell transduced with any of the recombinant viral genomes or expression vectors described herein is a heart cell, preferably a myocardial cell, of a rat, mouse, dog or a human, preferably of a mouse or a human, more preferably a human.

The provided host cells need not necessarily be present in an individual. The skilled person understands that introduction of the recombinant viral genomes and expression vectors as described herein may be performed in cell cultures. In some embodiments, the provided host cells are present in an artificial organ, preferably an artificial lung or heart. In some embodiments, the provided host cells are present in an organoid, preferably a lung or heart organoid. An "organoid" as defined herein is a miniaturized and simplified version of an organ produced *in vitro* in three dimensions that shows realistic micro-anatomy. The skilled person is able to arrive at such artificial organs and/or organoids using the host cells of the invention by applying generally known procedures in the art. The transduced host cells present in an artificial organ and/or organoid may be implanted to a vertebrate, preferably a mammal, more preferably a mouse, rat, dog or human, more preferably a mouse or human, most preferably a human, using generally known procedures in the art.

### Composition

In a further aspect there is provided a composition comprising a recombinant viral genome as described herein and/or an expression vector, preferably an adeno-associated viral vector, as described herein, optionally further comprising one or more pharmaceutically acceptable ingredients. Such a composition may be called a gene therapy composition. Preferably, the composition is a pharmaceutical composition. A composition such as a pharmaceutical composition may be formulated to be suitable for a particular mode of administration. Suitable administration modes are described elsewhere herein. The types of formulations, as well as formulation methods, that will be the most appropriate for a composition as described herein for it to be suitable for a particular mode of administration are well within the capabilities of the skilled person and are described in standard handbooks, such as Remington: The Science and Practice of Pharmacy, 23rd edition, Elsevier (2020), incorporated herein by reference in its entirety.

As used herein, "pharmaceutically acceptable ingredients" include pharmaceutically acceptable carriers, fillers, stabilizers, preservatives, solubilizers, vehicles, diluents and/or excipients. Accordingly, the one or more pharmaceutically acceptable ingredients may be selected from the group consisting of pharmaceutically acceptable carriers, fillers, stabilizers, preservatives, solubilizers, vehicles, diluents and/or excipients. Such pharmaceutically acceptable carriers, fillers, stabilizers, preservatives, solubilizers, vehicles, diluents and/or excipients may for instance be found in Remington: The Science and Practice of Pharmacy (supra).
A further compound may be present in a composition of the invention. Said compound may help in delivery of the composition. Examples of suitable compounds in this context are: compounds capable of forming complexes, nanoparticles, micelles and/or liposomes that deliver each constituent as described herein, complexed or trapped in a vesicle or liposome through a cell membrane. Many of these compounds are known in the art. Suitable compounds may comprise polyethylenimine (PEI), or similar cationic polymers, including polypropyleneimine or polyethylenimine copolymers (PECs) and derivatives; synthetic amphiphiles (SAINT-18); lipofectin^{™}, DOTAP. Additional examples of suitable compounds are inert carriers (such as calcium carbonate and sugars, for example sucrose, mannitol, lactose, or dextrose, and the like), solvents (such as water, saline, and the like), and propellant gases (such as chlorofluorocarbon (CFC), fluorocarban (FC), or hydrofluroalkane (HFA, and the like).

In some embodiments, a composition, preferably a pharmaceutical composition, is formulated to be suitable for a specific mode of administration of a recombinant viral genome as described herein and/or an expression vector, preferably an adeno-associated viral vector, as described herein, such as, but not limited to, intranasal, intratracheal, intrapulmonary administration or administration via inhalation. Non-limiting examples of exemplary formulations are aerosols, solutions and/or suspensions of particles in carriers which are suitable for respiratory tract and/or lung delivery, and dry powders.

In some embodiments, a composition, preferably a pharmaceutical composition, is comprised within a delivery device. In some embodiments, a composition, preferably a pharmaceutical composition is comprised within a delivery device suitable for delivery via inhalation (an inhalation device). Such devices are commonly known in the art, many being commercially available, and include inhalers (such as fixed dose inhalers, metered dose inhalers, dry powder inhalers, and the like) and nebulizers (such as jet nebulisers, ultrasonic nebulisers, mesh nebulisers, and the like). Nebulizers typically operate by aerosol particle generation. Exemplary nebulizers for delivering an aerosolized composition as described herein include the AERx^{™} (Aradigm), the Ultravent^{®} (Mallinkrodt), the Pari LC Plus^{™} or the Pari LC Star^{™} (Pari GmbH, Germany), the DeVilbiss Pulmo-Aide, and the Acorn II^{®} (Marquest Medical Products).

### Method and use

Also provided herein are recombinant viral genomes, expression vectors (preferably adeno-associated viral vectors) and compositions as described herein for use in therapy. In some embodiments, recombinant viral genomes, expression vectors (preferably adeno-associated viral vectors) and compositions as described herein are for use as a medicament.

The recombinant viral genomes, expression vectors (preferably adeno-associated viral vectors) and compositions described herein are particularly effective in treating and/or preventing conditions associated with shortened telomere length. A definition of "shortened telomere length" has been provided in the section entitled "general information". Accordingly, in a further aspect, the recombinant viral genomes, expression vectors (preferably adeno-associated viral vectors) and/or compositions described herein are provided for use in the treatment and/or prevention of a condition associated with shortened telomere length. In some embodiments, the recombinant viral genomes, expression vectors (preferably adeno-associated viral vectors) and/or compositions described herein are provided for use in the treatment and/or prevention of a lung condition associated with shortened telomere length. Non-limiting examples of such conditions are pulmonary fibrosis, chronic obstructive pulmonary disease (COPD), emphysema syndrome and conditions associated therewith. In some embodiments, the recombinant viral genomes, expression vectors (preferably adeno-associated viral vectors) and/or compositions described herein are provided for use in the treatment and/or prevention of a heart condition associated with shortened telomere length. Non-limiting examples of such conditions are coronary artery disease, peripheral artery disease, myocardial infarction and conditions associated therewith. In preferred embodiments, the recombinant viral genomes, expression vectors (preferably adeno-associated viral vectors) and/or compositions described herein are provided for use in the treatment and/or prevention of pulmonary fibrosis, myocardial infarction or conditions associated therewith.

Pulmonary fibrosis refers to a condition characterised by scarring of the lung tissue. Pulmonary fibrosis can be caused by many factors, including, but not limited to, lung cell telomere shortening, chronic inflammatory processes, infections, environmental compounds, ionizing radiation (for example radiation therapy to treat tumours of the chest), genetic predisposition, or chronic medical conditions (lupus, rheumatoid arthritis and others). Pulmonary fibrosis includes idiopathic pulmonary fibrosis (IPF), which refers to pulmonary fibrosis without an identifiable cause. Symptoms of pulmonary fibrosis include, but are not limited to, lung scarring, development of fibrotic volume, loss of lung volume, shortness of breath, dry cough, fatigue, weight loss, and nail clubbing. Conditions associated with pulmonary fibrosis include, but are not limited to, pulmonary hypertension, respiratory failure, pneumothorax, and lung cancer. In some embodiments, pulmonary fibrosis may be idiopathic pulmonary fibrosis (IPF).

Myocardial infarction refers to a condition characterized by tissue death (infarction) of the heart muscle (myocardium). Myocardial infarction can be caused by many factors, including, but not limited to, heart cell telomere shortening, atherosclerosis, high blood pressure (hypertension), hypercholesterolemia, diabetes, coronary heart disease, smoking, obesity, physical inactivity, environmental compounds, infections, genetic predisposition, or chronic inflammatory and other medical conditions. Symptoms of myocardial infarction include, but are not limited to, heart scarring, heart failure, an irregular heartbeat, fatigue, heart pain, cardiogenic shock or cardiac arrest. Conditions associated with myocardial infarction include, but are not limited to, myocardial infarction events (heart attack), tissue damage resulting from myocardial infarction (including loss of cardiomyocytes), fibrosis of the myocardium resulting from myocardial infarction, and reduced cardiac function resulting from myocardial infarction.

In some embodiments, recombinant viral genomes comprising a nucleotide sequence encoding a telomerase reverse transcriptase (TERT) operably linked to a lung-specific promoter, expression vectors (preferably adeno-associated viral vectors) comprising said genomes and/or compositions comprising said genomes and/or vectors as described herein are provided for use in the treatment and/or prevention of a lung condition associated with shortened telomere length, preferably of pulmonary fibrosis or a condition associated therewith. Preferably, the lung-specific promoter is an SpB promoter or a derivative thereof as described herein.

In some embodiments, recombinant viral genomes comprising a nucleotide sequence encoding a telomerase reverse transcriptase (TERT) operably linked to a heart-specific promoter, expression vectors (preferably adeno-associated viral vectors) comprising said genomes and/or compositions comprising said genomes and/or vectors as described herein are provided for use in the treatment and/or prevention of a heart condition associated with shortened telomere length, preferably of myocardial infarction or a condition associated therewith. Preferably, the heart-specific promoter is a CMVenh-MLC2v promoter, a troponin promoter or a derivative thereof, preferably a troponin promoter or derivative thereof, as described herein.

In a further aspect, there is provided a method of treatment and/or prevention of a condition associated with shortened telomere length, comprising administering a therapeutically effective amount of a recombinant viral genome, an expression vector (preferably an adeno-associated viral vector) and/or composition described herein to a subject such as a subject in need thereof. In some embodiments, the condition associated with shortened telomere length is a lung condition, preferably it is pulmonary fibrosis or a condition associated therewith. In some embodiments, the condition associated with shortened telomere length is a heart condition, preferably it is myocardial infarction or a condition associated therewith.

In some embodiments, there is provided a method of treatment and/or prevention of a lung condition associated with shortened telomere length, preferably of pulmonary fibrosis or a condition associated therewith, comprising administering a therapeutically effective amount of a recombinant viral genome comprising a nucleotide sequence encoding a telomerase reverse transcriptase (TERT) operably linked to a lung-specific promoter, an expression vector (preferably an adeno-associated viral vector) comprising said genome and/or a composition comprising said genome and/or vector as described herein to a subject such as a subject in need thereof. Preferably, the lung-specific promoter is an SpB promoter or a derivative thereof as described herein.

In some embodiments, there is provided a method of treatment and/or prevention of a heart condition associated with shortened telomere length, preferably of myocardial infarction or a condition associated therewith, comprising administering a therapeutically effective amount of a recombinant viral genome comprising a nucleotide sequence encoding a telomerase reverse transcriptase (TERT) operably linked to a heart-specific promoter, an expression vector (preferably an adeno-associated viral vector) comprising said genome and/or a composition comprising said genome and/or vector as described herein to a subject such as a subject in need thereof. Preferably, the heart-specific promoter is a CMVenh-MLC2v promoter, a troponin promoter or a derivative thereof, preferably a troponin promoter or derivative thereof, as described herein.

As used herein, an "effective amount" is an amount sufficient to exert beneficial or desired results. Accordingly, a "therapeutically effective amount" is an amount that, when administered to a subject in need thereof, is sufficient to exert some therapeutic effect as described herein, such as, but not limited to, a reduction in the magnitude of at least one symptom and/or the improvement of at least one parameter of a condition associated with shortened telomere length, preferably of pulmonary fibrosis, myocardial infarction or a condition associated therewith as described elsewhere herein. An amount that is "therapeutically effective" will vary from subject to subject, depending on the age, the disease progression and overall general condition of the individual. An appropriate "therapeutically effective" amount in any individual case may be determined by the skilled person using routine experimentation, such as the methods described later herein, and/or the methods of the experimental part herein. A "subject in need" may be any individual affected by, and/or at risk of developing, a condition associated with shortened telomere length

In a further aspect there is provided a use of a recombinant viral genome, an expression vector (preferably an adeno-associated viral vector) or a composition as described herein, for the manufacture of a medicament for the treatment and/or prevention of a condition associated with shortened telomere length. In some embodiments, the condition associated with shortened telomere length is a lung condition, preferably it is pulmonary fibrosis or a condition associated therewith. In some embodiments, the condition associated with shortened telomere length is a heart condition, preferably it is myocardial infarction or a condition associated therewith.

In some embodiments, there is provided a use of a recombinant viral genome comprising a nucleotide sequence encoding a telomerase reverse transcriptase (TERT) operably linked to a lung-specific promoter, an expression vector (preferably an adeno-associated viral vector) comprising said genome and/or a composition comprising said genome and/or vector as described herein for the manufacture of a medicament for the treatment and/or prevention of a lung condition associated with shortened telomere length, preferably of pulmonary fibrosis or a condition associated therewith. Preferably, the lung-specific promoter is an SpB promoter or a derivative thereof as described herein.

In some embodiments, there is provided a use of a recombinant viral genome comprising a nucleotide sequence encoding a telomerase reverse transcriptase (TERT) operably linked to a heart-specific promoter, an expression vector (preferably an adeno-associated viral vector) comprising said genome and/or a composition comprising said genome and/or vector as described herein for the manufacture of a medicament for the treatment and/or prevention of a heart condition associated with shortened telomere length, preferably of myocardial infarction or a condition associated therewith. Preferably, the heart-specific promoter is a CMVenh-MLC2v promoter, a troponin promoter or a derivative thereof, preferably a troponin promoter or derivative thereof, as described herein.

In a further aspect there is provided a use of a recombinant viral genome, an expression vector, preferably an adeno-associated viral vector, or a composition as described herein, for the treatment and/or prevention of a condition associated with shortened telomere length. In some embodiments, the condition associated with shortened telomere length is a lung condition, preferably it is pulmonary fibrosis or a condition associated therewith. In some embodiments, the condition associated with shortened telomere length is a heart condition, preferably it is myocardial infarction or a condition associated therewith.

In some embodiments, there is provided a use of a recombinant viral genome comprising a nucleotide sequence encoding a telomerase reverse transcriptase (TERT) operably linked to a lung-specific promoter, an expression vector (preferably an adeno-associated viral vector) comprising said genome and/or a composition comprising said genome and/or vector as described herein for the treatment and/or prevention of a lung condition associated with shortened telomere length, preferably of pulmonary fibrosis or a condition associated therewith. Preferably, the lung-specific promoter is an SpB promoter or a derivative thereof as described herein.

In some embodiments, there is provided a use of a recombinant viral genome comprising a nucleotide sequence encoding a telomerase reverse transcriptase (TERT) operably linked to a heart-specific promoter, an expression vector (preferably an adeno-associated viral vector) comprising said genome and/or a composition comprising said genome and/or vector as described herein for the treatment and/or prevention of a heart condition associated with shortened telomere length, preferably of myocardial infarction or a condition associated therewith. Preferably, the heart-specific promoter is a CMVenh-MLC2v promoter, a troponin promoter or a derivative thereof, preferably a troponin promoter or derivative thereof, as described herein.

Within the context of recombinant viral genome for use, expression vectors (preferably adeno-associated viral vectors) for use, compositions for use, methods and uses according to the invention, the therapy and/or medicament may involve expression, preferably specific expression, of TERT in the lungs. Within the context of recombinant viral genome for use, expression vectors (preferably adeno-associated viral vectors) for use, compositions for use, methods and uses according to the invention, the therapy and/or medicament may involve expression, preferably specific expression of TERT in the heart. A description of expression has been provided in the section entitled "general information". In some embodiments, expression of TERT does not involve expression in at least one, at least two, at least three, at least four organs selected from the group consisting of liver, CNS, brain, adipose tissue (white and/or brown), skeletal muscle, heart, kidney, colon, hematopoietic tissue, lung, ovary, spleen, stomach, pancreas, testis, epididymis, intestine, and others.

In preferred embodiments, a treatment or a therapy or a use or the administration of a medicament as described herein does not have to be repeated. In some embodiments, a treatment or a therapy or a use or the administration of a medicament as described herein may be repeated each year or each 2, 3, 4, 5, 6, 7, 8, 9 or 10 years, including intervals between any two of the listed values.

The subject treated may be a vertebrate, preferably a mammal such as a cat, a mouse, a rat, a dog, or a human. In preferred embodiments, the subject treated is a human. Treatment administration may be performed to an individual, a cell, tissue, and/or an organ of an individual affected and/or at risk of developing a condition associated with shortened telomere length, preferably pulmonary fibrosis, myocardial infarction or conditions associated therewith. Treatment administration may be performed directly or indirectly *in vivo, ex vivo* or *in vitro,* using suitable means known in the art. Improvements in means for providing an individual or a cell, tissue, and/or organ of said individual with a recombinant viral genome and/or an expression vector (preferably an adeno-associated viral vector) and/or a composition of the invention are anticipated, considering the progress that has already thus far been achieved. Such future improvements may of course be incorporated to achieve the mentioned effect of the invention. Depending on the disease or condition, a cell, tissue and/or organ of said individual may be as earlier described herein. When administering a recombinant viral genome and/or an expression vector, preferably an adeno-associated viral vector, and/or a composition of the invention, it is preferred that such gene construct and/or an expression vector and/or a composition is dissolved in a solution that is compatible with the delivery method. Such solutions are generally known in the art, see for example Remington: The Science and Practice of Pharmacy (supra). Treatment administration may be performed using different administration modes which are generally known in the art. An administration mode may be intravenous, intranasal, intramuscular, intraperitoneal, via inhalation, intraparenchymal, subcutaneous, intraarticular, intra-adipose tissue, oral, intrahepatic, intrasplanchnic, intra-ear, intrathoracic, intrapulmonary, intracardial, transendocardial or intratracheal administration. Preferred administration modes for lung-specific expression are via inhalation, intranasal, intratracheal, intrapulmonary, and intravenous administration. "Intratracheal administration" or "intratracheal instillation" refers to direct administration into the trachea. "Intravenous administration" refers to direct administration into a vein, typically by injection. A preferred administration mode for heart-specific administration is intracardial, transendocardial or intravenous administration. "Intracardial administration" refers to direct administration into the heart muscle or ventricles "Transendocardial administration" refers to direct administration into the endocardium.

In some embodiments, a treatment or a therapy or a use or the administration of a medicament for a treatment as described herein exhibit at least one, at least two, at least three, or all of the following benefits over known treatments, therapies, uses or administration of medicaments:
- Increased TERT expression in target tissues
- Decreased TERT expression in off-target tissues
- Prevention of formation of intermediate species comprising truncated viral genomes associated with the utilization of viral vectors with low encapsidation capacity
- Increased therapeutic efficacy

In some embodiments, a treatment or a therapy or a use or the administration of a medicament for a treatment as described herein alleviates, removes, prevents and/or reverses shortening of telomeres.
"Shortened telomeres" or "short telomeres" generally refers to telomeres below a certain length, e.g. 8 kb, 7 kb, 6kb, 5kb, or shorter. The skilled person understands that telomere length depends not only on whether an individual is healthy, but also on the individual's age. Typically, shortened telomeres have a length that is below the 20th or 10^{th} percentile of the telomere length of a population of healthy individuals belonging to a certain age group, which indicates the telomere length below which 20% or 10% of the telomeres fall. Conversely, long telomeres typically have a length that is above the 80th or 90th percentile of the telomere length of a population of healthy individuals belonging to a certain age group. Telomere length may be assessed in samples taken directed from a treated individual, or a cell, tissue, and/or organ of a treated individual, according to generally known methods in the art. For example, standard hybridization techniques, such as fluorescence *in situ* hybridization (FISH), quantitative fluorescent *in situ* hybridization (Q-FISH), or high throughput quantitative fluorescent *in situ* hybridization (HT Q-FISH) as described in Gonzalez-Suarez et al. (2001) EMBO J 20(11): 2619-30, incorporated by reference herein in its entirety, may be used. Alternatively, telomere length may be measured as described in any one of the disclosures of WO2016/020346, Canela et al. (2007) Methods Mol Biol 371: 45-72, or Bär et al. (2018) Nat Comm 5: 5863, incorporated by reference herein in their entireties. Telomere length may also be inferred by monitoring the deterioration of or the improvement in a symptom and/or a parameter of a condition associated with shortened telomere length as discussed elsewhere herein using standard procedures in the art, as for example provided in the Examples section herein.
Samples may be taken throughout the course of the treatment so that both absolute telomere length and the rate of telomere lengthening or shortening over the course of treatment can be determined. Samples may be taken every day during the course of treatment, or at longer intervals. In some embodiments, samples are taken once a week, once every two weeks, once every three weeks, once every four weeks, once every five weeks, once every six weeks or at longer intervals. Comparisons of telomere length can be made by comparing the proportion of shortened telomeres in the taken samples. The proportion of shortened telomeres may be assessed by measuring the fraction of telomeres presenting an intensity below the mean intensity of the sample as measured by an *in situ* hybridization technique, such as FISH or Q-FISH. In some embodiments, the proportion of shortened telomeres is the fraction of telomeres presenting an intensity 75%, 70%, 65%, 60%, 55%, 50%, 45%, 40% or lower as compared to the mean intensity of the sample. In some embodiments, the proportion of shortened telomeres in a sample taken directed from a treated individual, or a cell, tissue, and/or organ of a treated individual, is decreased by 10%, 20%, 30%, 40%, 50%, 60%, 70%, or greater as compared to a control sample. A control sample may be taken from the individual to be treated, or a cell, tissue, and/or organ of an individual to be treated prior to commencement of the treatment and/or from an untreated individual suffering from the same condition, or a cell, tissue, and/or organ of an untreated individual suffering from the same condition.

In some embodiments, a treatment or a therapy or a use or the administration of a medicament for the treatment of a pulmonary fibrosis or a condition associated therewith alleviates, removes and/or prevents a symptom and/or improves a parameter associated therewith selected from the group consisting of lung scarring, development of fibrotic volume, loss of lung volume, shortness of breath, dry cough, fatigue, weight loss, and nail clubbing, preferably development of fibrotic volume and/or loss of lung volume.

In some embodiments, a treatment or a therapy or a use or the administration of a medicament for the treatment of a myocardial infarction or a condition associated therewith alleviates, removes and/or prevents a symptom and/or improves a parameter associated therewith selected from the group consisting of heart scarring, heart failure, an irregular heartbeat, fatigue, heart pain, cardiogenic shock or cardiac arrest, preferably heart scarring.

Alleviating a symptom of a condition as discussed herein may mean that said symptom is improved or decreased or that the progression of a typical symptom has been slowed down in an individual, in a cell, tissue or organ of said individual as assessed by a physician. A decrease or improvement of a typical symptom may mean a slowdown in progression of symptom development or a complete disappearance of symptoms. Symptoms, and thus also a decrease in symptoms, can be assessed using a variety of methods, to a large extent the same methods as used in diagnosis of the relevant conditions, including clinical examination and routine laboratory tests. Laboratory tests may include both macroscopic and microscopic methods, molecular methods, radiographic methods such as X-rays, CT-scans, spirometry, biochemical methods, immunohistochemical methods and others. In this context, "decrease" (respectively "improvement") means at least a detectable decrease (respectively a detectable improvement) using an assay known to a person of skill in the art, such as assays as carried out in the experimental part. The decrease may be a decrease of at least 5%, at least 10%, at least 20%, at least 30%, at least 40%, at least 50%, at least 60%, at least 70%, at least 80%, at least 90% or at least 100%. The decrease may be seen after at least one week, one month, six months, one year or more of treatment using a recombinant viral genome and/or an expression vector (preferably an adeno-associated viral vector) and/or a composition of the invention. Preferably, the decrease is observed after a single administration. In some embodiments, the decrease is observed for a duration of at least one week, one month, six months, 1 year, 2 years, 3 years, 4 years, 5 years, 6 years, 7 years, 8 years, 9 years, 10 years, 12 years, 15 years, 20 years or more, preferably after a single administration.

Improving a parameter may mean that the value of a typical parameter associated with the relevant disease (e.g. lung volume in the case of pulmonary fibrosis) is improved in an individual, in a cell, tissue or organ of said individual as assessed by a physician. In this context, improvement of a parameter may be interpreted as to mean that said parameter assumes a value closer to the value displayed by a healthy individual. The improvement of a parameter may be seen after at least one week, one month, six months, one year or more of treatment using a recombinant viral vector and/or an expression vector, preferably an adeno-associated viral vector, and/or a composition of the invention. Preferably, the improvement is observed after a single administration. In some embodiments, the improvement is observed for a duration of at least one week, one month, six months, 1 year, 2 years, 3 years, 4 years, 5 years, 6 years, 7 years, 8 years, 9 years, 10 years, 12 years, 15 years, 20 years or more, preferably after a single administration.

In some embodiments, a treatment or a therapy or a use or the administration of a medicament for treatment as described herein results in an increase in overall physical fitness of a treated individual as compared to the same individual prior to commencement of the treatment or an untreated individual suffering from the same condition. Overall physical fitness may be determined by evaluating and/or measuring physical attributes associated with the relevant condition as assessed by a physician and an increase in overall physical fitness may mean that at least one such attribute is improved. Examples of physical attributes include dry cough, fatigue, and others.

In some embodiments, a treatment or a therapy or a use or the administration of a medicament for treatment as described herein results in an increase in the lifespan of a treated individual as compared to an untreated individual suffering from the same condition. The extension of said lifespan may depend on the relevant condition to be treated and preferably is 5%, 10%, 15%, 20% or more.

### General information

Unless stated otherwise, all technical and scientific terms used herein have the same meaning as customarily and ordinarily understood by a person of ordinary skill in the art to which this invention belongs, and read in view of this disclosure.

### Lung and heart

The terms "lung" and "heart" as used herein refer to the organs of the respiratory and cardiovascular system, respectively, as customarily and ordinarily understood by the skilled person. "Epithelial" cell refers to a cell of the epithelial tissue, as customarily and ordinarily understood by the skilled person. "Alveolar epithelial" refers to cell of the epithelial tissue of the alveoli , as customarily and ordinarily understood by the skilled person. A "myocardial cell" refers to a cell of the myocardium, as customarily and ordinarily understood by the skilled person.

### Sequence identity

In the context of the invention, a nucleotide sequence such as a nucleotide sequence encoding a TERT is represented by a nucleic acid or nucleotide sequence which encodes a protein fragment or a polypeptide or a peptide or a derived peptide. In the context of the invention, a TERT, a TERT fragment, fragment or a polypeptide or a peptide or a derived peptide is represented by an amino acid sequence.

It is to be understood that each nucleic acid molecule or protein fragment or polypeptide or peptide or derived peptide or construct as identified herein by a given sequence identity number (SEQ ID NO) is not limited to this specific sequence as disclosed. Each coding sequence as identified herein encodes a given protein fragment or polypeptide or peptide or derived peptide or construct or is itself a protein fragment or polypeptide or construct or peptide or derived peptide.

Throughout this application, each time one refers to a specific nucleotide sequence SEQ ID NO (take SEQ ID NO: X as example) encoding a given protein fragment or polypeptide or peptide or derived peptide, one may replace it by:
i. a nucleotide sequence comprising a nucleotide sequence that has at least 60%, 70%, 80%, 90%, 95% or 99% sequence identity with SEQ ID NO: X;
ii. a nucleotide sequence the sequence of which differs from the sequence of a nucleic acid molecule of (i) due to the degeneracy of the genetic code; or
iii. a nucleotide sequence that encodes an amino acid sequence that has at least 60%, 70%, 80%, 90%, 95% or 99% amino acid identity or similarity with an amino acid sequence encoded by a nucleotide sequence SEQ ID NO: X.

Another preferred level of sequence identity or similarity is 70%. Another preferred level of sequence identity or similarity is 80%. Another preferred level of sequence identity or similarity is 90%. Another preferred level of sequence identity or similarity is 95%. Another preferred level of sequence identity or similarity is 99%.

Throughout this application, each time one refers to a specific amino acid sequence SEQ ID NO (take SEQ ID NO: Y as example), one may replace it by: a polypeptide represented by an amino acid sequence comprising a sequence that has at least 60%, 70%, 80%, 90%, 95% or 99% sequence identity or similarity with amino acid sequence SEQ ID NO: Y. Another preferred level of sequence identity or similarity is 70%. Another preferred level of sequence identity or similarity is 80%. Another preferred level of sequence identity or similarity is 90%. Another preferred level of sequence identity or similarity is 95%. Another preferred level of sequence identity or similarity is 99%.

Each nucleotide sequence or amino acid sequence described herein by virtue of its identity or similarity percentage with a given nucleotide sequence or amino acid sequence respectively has in a further preferred embodiment an identity or a similarity of at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% with the given nucleotide or amino acid sequence, respectively.
Each non-coding nucleotide sequence (i.e. of a promoter or of another regulatory region) could be replaced by a nucleotide sequence comprising a nucleotide sequence that has at least 60% sequence identity or similarity with a specific nucleotide sequence SEQ ID NO (take SEQ ID NO: A as example). A preferred nucleotide sequence has at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100% identity with SEQ ID NO: A. In a preferred embodiment, such non-coding nucleotide sequence such as a promoter exhibits or exerts at least an activity of such a non-coding nucleotide sequence such as an activity of a promoter as known to a person of skill in the art.

The terms "homology", "sequence identity" and the like are used interchangeably herein. Sequence identity is described herein as a relationship between two or more amino acid (polypeptide or protein) sequences or two or more nucleic acid (polynucleotide) sequences, as determined by comparing the sequences. In a preferred embodiment, sequence identity is calculated based on the full length of two given SEQ ID NO's or on a part thereof. Part thereof preferably means at least 50%, 60%, 70%, 80%, 90%, or 100% of both SEQ ID NO's. In the art, "identity" also refers to the degree of sequence relatedness between amino acid or nucleic acid sequences, as the case may be, as determined by the match between strings of such sequences. "Similarity" between two amino acid sequences is determined by comparing the amino acid sequence and its conserved amino acid substitutes of one polypeptide to the sequence of a second polypeptide. "Identity" and "similarity" can be readily calculated by known methods, including but not limited to those described in Bioinformatics and the Cell: Modern Computational Approaches in Genomics, Proteomics and transcriptomics, Xia X., Springer International Publishing, New York, 2018; and Bioinformatics: Sequence and Genome Analysis, Mount D., Cold Spring Harbor Laboratory Press, New York, 2004, each incorporated by reference herein in its entirety.
"Sequence identity" and "sequence similarity" can be determined by alignment of two peptide or two nucleotide sequences using global or local alignment algorithms, depending on the length of the two sequences. Sequences of similar lengths are preferably aligned using a global alignment algorithm (e.g. Needleman-Wunsch) which aligns the sequences optimally over the entire length, while sequences of substantially different lengths are preferably aligned using a local alignment algorithm (e.g. Smith-Waterman). Sequences may then be referred to as "substantially identical" or "essentially similar" when they (when optimally aligned by for example the program EMBOSS needle or EMBOSS water using default parameters) share at least a certain minimal percentage of sequence identity (as described below).

A global alignment is suitably used to determine sequence identity when the two sequences have similar lengths. When sequences have a substantially different overall length, local alignments, such as those using the Smith-Waterman algorithm, are preferred. EMBOSS needle uses the Needleman-Wunsch global alignment algorithm to align two sequences over their entire length (full length), maximizing the number of matches and minimizing the number of gaps. EMBOSS water uses the Smith-Waterman local alignment algorithm. Generally, the EMBOSS needle and EMBOSS water default parameters are used, with a gap open penalty = 10 (nucleotide sequences) / 10 (proteins) and gap extension penalty = 0.5 (nucleotide sequences) / 0.5 (proteins). For nucleotide sequences the default scoring matrix used is DNAfull and for proteins the default scoring matrix is Blosum62 (Henikoff & Henikoff, 1992, PNAS 89, 915-919, incorporated herein by reference in its entirety).

Alternatively, percentage similarity or identity may be determined by searching against public databases, using algorithms such as FASTA, BLAST, etc. Thus, the nucleic acid and protein sequences of some embodiments of the present invention can further be used as a "query sequence" to perform a search against public databases to, for example, identify other family members or related sequences. Such searches can be performed using the BLASTn and BLASTx programs (version 2.0) of Altschul, et al. (1990) J. Mol. Biol. 215:403-10, incorporated herein by reference in its entirety. BLAST nucleotide searches can be performed with the NBLAST program, score = 100, wordlength = 12 to obtain nucleotide sequences homologous to oxidoreductase nucleic acid molecules of the invention. BLAST protein searches can be performed with the BLASTx program, score = 50, wordlength = 3 to obtain amino acid sequences homologous to protein molecules of the invention. To obtain gapped alignments for comparison purposes, Gapped BLAST can be utilized as described in Altschul et al., (1997) Nucleic Acids Res. 25(17): 3389-3402, incorporated herein by reference in its entirety. When utilizing BLAST and Gapped BLAST programs, the default parameters of the respective programs (e.g., BLASTx and BLASTn) can be used. See the homepage of the National Center for Biotechnology Information accessible on the world wide web at www.ncbi.nlm.nih.gov/.

Optionally, in determining the degree of amino acid similarity, the skilled person may also take into account so-called conservative amino acid substitutions. As used herein, "conservative" amino acid substitutions refer to the interchangeability of residues having similar side chains. Examples of classes of amino acid residues for conservative substitutions are given in the Tables below.

| | |
|---|---|
| Acidic Residues | Asp (D) and Glu (E) |
| Basic Residues | Lys (K), Arg (R), and His (H) |
| Hydrophilic Uncharged Residues | Ser (S), Thr (T), Asn (N), and Gln (Q) |
| Aliphatic Uncharged Residues | Gly (G), Ala (A), Val (V), Leu (L), and Ile (I) |
| Non-polar Uncharged Residues | Cys (C), Met (M), and Pro (P) |
| Aromatic Residues | Phe (F), Tyr (Y), and Trp (W) |

Alternative conservative amino acid residue substitution classes :

| | | | |
|---|---|---|---|
| 1 | A | S | T |
| 2 | D | E | |
| 3 | N | Q | |
| 4 | R | K | |
| 5 | I | L | M |
| 6 | F | Y | W |

Alternative physical and functional classifications of amino acid residues:

| | |
|---|---|
| Alcohol group-containing residues | S and T |
| Aliphatic residues | I, L, V, and M |
| Cycloalkenyl-associated residues | F, H, W, and Y |
| Hydrophobic residues | A, C, F, G, H, I, L, M, R, T, V, W, and Y |
| Negatively charged residues | D and E |
| Polar residues | C, D, E, H, K, N, Q, R, S, and T |
| Positively charged residues | H, K, and R |
| Small residues | A, C, D, G, N, P, S, T, and V |
| Very small residues | A, G, and S |
| Residues involved in turn formation | A, C, D, E, G, H, K, N, Q, R, S, P and T |
| Flexible residues | Q, T, K, S, G, P, D, E, and R |

For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulphur-containing side chains is cysteine and methionine. Preferred conservative amino acids substitution groups are: valine-leucine-isoleucine, phenylalanine-tyrosine, lysine-arginine, alanine-valine, and asparagine-glutamine. Substitutional variants of the amino acid sequence disclosed herein are those in which at least one residue in the disclosed sequences has been removed and a different residue inserted in its place. Preferably, the amino acid change is conservative. Preferred conservative substitutions for each of the naturally occurring amino acids are as follows: Ala to Ser; Arg to Lys; Asn to Gin or His; Asp to Glu; Cys to Ser or Ala; Gin to Asn; Glu to Asp; Gly to Pro; His to Asn or Gin; lie to Leu or Val; Leu to lie or Val; Lys to Arg; Gin or Glu; Met to Leu or lie; Phe to Met, Leu or Tyr; Ser to Thr; Thr to Ser; Trp to Tyr; Tyr to Trp or Phe; and, Val to lie or Leu.

### Gene or coding nucleotide sequence

The term "gene" means a DNA fragment comprising a region (transcribed region), which is transcribed into an RNA molecule (e.g. an mRNA) in a cell, operably linked to suitable regulatory regions (e.g. a promoter). A gene will usually comprise several operably linked fragments, such as a promoter, a 5' leader sequence, a coding region and a 3'-untranslated region (3'-end) e.g. comprising a polyadenylation- and/or transcription termination site. A chimeric or recombinant gene (such as a TERT gene) is a gene not normally found in nature, such as a gene in which for example the promoter is not associated in nature with part or all of the transcribed DNA region. "Expression of a gene" refers to the process wherein a DNA region which is operably linked to appropriate regulatory regions, particularly a promoter, is transcribed into an RNA, which is biologically active, i.e. which is capable of being translated into a biologically active protein or peptide.

In the context of the invention, a TERT gene may normally not be expressed or expressed at an insufficient level in a cell. In this context, "insufficient" means that although said TERT gene is expressed in a cell, a condition and/or disease as described herein could still be developed. In this case, the invention allows the over-expression of TERT. Coding nucleotide sequences may comprise sequences that are native to the cell, sequences that naturally do not occur in the cell and it may comprise combinations of both. A gene may contain sequences encoding a TERT and/or additional proteins as earlier identified herein that may be operably linked to appropriate regulatory sequences for expression of the sequences encoding a TERT in the cell. Preferably, the introduced gene is not integrated into the host cell's genome.

### Promoter

As used herein, the term "promoter" or "transcription regulatory sequence" refers to a nucleic acid fragment that functions to control the transcription of one or more coding sequences, and is located upstream with respect to the direction of transcription of the transcription initiation site of the coding sequence, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter. A "constitutive" promoter is a promoter that is active in most tissues under most physiological and developmental conditions. An "inducible" promoter is a promoter that is physiologically or developmentally regulated, e.g. by the application of a chemical inducer. As used herein, a "regulator" or "transcriptional regulator" is a protein that controls the rate of transcription of genetic information from DNA to messenger RNA, by binding to a specific DNA sequence. Expression may be assessed as described elsewhere in this section. "Transcriptional initiation capability" or "promoter strength" refers to the extent of the capability of a promoter to initiate transcription of a coding nucleotide sequence to which it is operably linked.

### Operably linked

As used herein, the term "operably linked" refers to a linkage of polynucleotide elements in a functional relationship. A nucleic acid is "operably linked" when it is placed into a functional relationship with another nucleic acid sequence. For instance, a transcription regulatory sequence is operably linked to a coding sequence if it affects the transcription of the coding sequence. Operably linked means that the DNA sequences being linked are typically contiguous and, where necessary to join two protein encoding regions, contiguous and in reading frame. Linking can be accomplished by ligation at convenient restriction sites or at adapters or linkers inserted in lieu thereof, or by gene synthesis.

### Proteins and amino acids

The terms "protein" or "polypeptide" or "amino acid sequence" are used interchangeably and refer to molecules consisting of a chain of amino acids, without reference to a specific mode of action, size, 3-dimensional structure or origin. In amino acid sequences as described herein, amino acids or "residues" are denoted by three-letter symbols. These three-letter symbols as well as the corresponding one-letter symbols are well known to a person of skill in the art and have the following meaning: A (Ala) is alanine, C (Cys) is cysteine, D (Asp) is aspartic acid, E (Glu) is glutamic acid, F (Phe) is phenylalanine, G (Gly) is glycine, H (His) is histidine, I (lie) is isoleucine, K (Lys) is lysine, L (Leu) is leucine, M (Met) is methionine, N (Asn) is asparagine, P (Pro) is proline, Q (Gin) is glutamine, R (Arg) is arginine, S (Ser) is serine, T (Thr) is threonine, V (Val) is valine, W (Trp) is tryptophan, Y (Tyr) is tyrosine. A residue may be any proteinogenic amino acid, but also any non-proteinogenic amino acid such as D-amino acids and modified amino acids formed by post-translational modifications, and also any non-natural amino acid.

### Expression vector

The phrase "expression vector" or "vector" or "delivery vector" generally refers to a tool in molecular biology used to obtain gene expression in a cell, for example by introducing a nucleotide sequence that is capable of effecting expression of a gene or a coding sequence in a host compatible with such sequences. An expression vector may carry a genome that is able to stabilize and remain episomal in a cell. Within the context of the invention, a cell may mean to encompass a cell used to make the vector or a cell wherein the vector will be administered. Alternatively, a vector is capable of integrating into a cell's genome, for example through homologous recombination or otherwise.

### Viral vector

A viral vector or a viral expression vector a viral gene therapy vector generally refers to a virus-derived vector used in molecular biology to obtain gene expression in a cell, for example by introducing a nucleotide sequence that is capable of effecting expression of a gene or a coding sequence in a host compatible with such sequences.

A viral vector or a viral gene therapy vector is a vector that is suitable for gene therapy. Vectors that are suitable for gene therapy are described in Anderson 1998, Nature 392: 25-30; Walther and Stein, 2000, Drugs 60: 249-71; Kay et al., 2001, Nat. Med. 7: 33-40; Russell, 2000, J. Gen. Virol. 81: 2573-604; Amado and Chen, 1999, Science 285: 674-6; Federico, 1999, Curr. Opin. Biotechnol.10: 448-53; Vigna and Naldini, 2000, J. Gene Med. 2: 308-16; Marin et al., 1997, Mol. Med. Today 3: 396-403; Peng and Russell, 1999, Curr. Opin. Biotechnol. 10: 454-7; Sommerfelt, 1999, J. Gen. Virol. 80: 3049-64; Reiser, 2000, Gene Ther. 7: 910-3; WO2016/020346, WO2016/020345, Bär et al, 2018, Nat. Comm. 5: 5863, van Lieshout et al, 2018, Mol. Ther. Meth. Clin. Dev. 9, and references cited therein; all of which are incorporated herein by reference in their entirety.

A particularly suitable gene therapy vector includes an adenoviral and adeno-associated virus (AAV) vector. These vectors infect a wide number of dividing and non-dividing cell types including synovial cells and liver cells. The episomal nature of the adenoviral and AAV vectors after cell entry makes these vectors suited for therapeutic applications, (Russell, 2000, J. Gen. Virol. 81: 2573-2604; Goncalves, 2005, Virol J. 2(1):43; WO2016/020346; WO2016/020345; Bär et al, 2018, Nat. Comm. 5: 5863; van Lieshout et al, 2018, Mol. Ther. Meth. Clin. Dev. 9, incorporated herein by reference in their entirety) as indicated above. AAV vectors are even more preferred since they are known to result in very stable long-term expression of transgene expression (up to 9 years in dog (Niemeyer et al, Blood. 2009 Jan 22;113(4):797-806) and ∼ 10 years in human (Buchlis, G. et al., Blood. 2012 Mar 29;119(13):3038-41). Preferred adenoviral vectors are modified to reduce the host response as reviewed by Russell (2000, supra). Methods for gene therapy using AAV vectors are described by Wang et al., 2005, J Gene Med. March 9 (Epub ahead of print), Mandel et al., 2004, Curr Opin Mol Ther. 6(5):482-90, and Martin et al., 2004, Eye 18(11):1049-55, Nathwani et al, N Engl J Med. 2011 Dec 22;365(25):2357-65, Apparailly et al, Hum Gene Ther. 2005 Apr;16(4):426-34; WO2016/020346; WO2016/020345; Bär et al, 2018, Nat. Comm. 5: 5863; van Lieshout et al, 2018, Mol. Ther. Meth. Clin. Dev. 9, all of which are incorporated herein by reference in their entirety.

Another suitable gene therapy vector includes a retroviral vector. A preferred retroviral vector for application in the present invention is a lentiviral based expression construct. Lentiviral vectors have the ability to infect and to stably integrate into the genome of dividing and non-dividing cells (Amado and Chen, 1999 Science 285: 674-6, incorporated herein by reference in their entirety). Methods for the construction and use of lentiviral based expression constructs are described in U.S. Patent No.'s 6,165,782, 6,207,455, 6,218,181, 6,277,633 and 6,323,031 and in Federico (1999, Curr Opin Biotechnol 10: 448-53) and Vigna et al. (2000, J Gene Med 2000; 2: 308-16); all of which are incorporated herein by reference in their entirety. Other suitable gene therapy vectors include an adenovirus vector, a herpes virus vector, a polyoma virus vector or a vaccinia virus vector.

### Adeno-associated virus vector (AAV vector)

The terms "adeno-associated virus", "AAV virus", "AAV virion", "AAV viral particle" and "AAV particle", used as synonyms herein, refer to a viral particle composed of at least one capsid protein of AAV (preferably composed of all capsid protein of a particular AAV serotype) and an encapsulated polynucleotide of the AAV genome. If the particle comprises a heterologous polynucleotide (i.e. a polynucleotide different from a wild-type AAV genome, such as a gene or a recombinant viral genome to be delivered to a mammalian cell) flanked by AAV inverted terminal repeats, then they are typically known as a "AAV vector particle" or "AAV viral vector" or "AAV vector". AAV refers to a virus that belongs to the genus *Dependoparvovirus* (also referred to as *Dependovirus*) of the family *Parvoviridae.* The AAV genome is approximately 4.7 kb in length and it consists of single strand deoxyribonucleic acid (ssDNA) that can be positive or negative detected. The invention also encompasses the use of double stranded AAV also called dsAAV or scAAV. The genome includes inverted terminal repeats (ITR) at both ends of the DNA strand, and two open reading frames (ORFs): rep and cap. The frame rep is made of four overlapping genes that encode proteins Rep necessary for AAV lifecycle. The frame cap contains nucleotide sequences overlapping with capsid proteins: VP1, VP2 and VP3, which interact to form a capsid of icosahedral symmetry (see Carter and Samulski ., 2000, and Gao et al, 2004, incorporated for reference herein in their entirety).

A preferred viral vector or a preferred gene therapy vector is an AAV vector. An AAV vector as used herein preferably comprises a recombinant AAV vector (rAAV vector). A "rAAV vector" as used herein refers to a recombinant vector comprising part of an AAV genome encapsidated in a protein shell of capsid protein derived from an AAV serotype as explained herein. Part of an AAV genome may contain the inverted terminal repeats (ITR) derived from an adeno-associated virus serotype, such as AAV1, AAV2, AAV3, AAV4, AAV5 and others, as described elsewhere herein.

Protein shell comprised of capsid protein may be derived from any AAV serotype. A protein shell may also be named a capsid protein shell. rAAV vector may have one or preferably all wild type AAV genes deleted, but may still comprise functional ITR nucleic acid sequences. Functional ITR sequences are necessary for the replication, rescue and packaging of AAV virions. The ITR sequences may be wild type sequences or may have at least 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity with wild type sequences or may be altered by for example by insertion, mutation, deletion or substitution of nucleotides, as long as they remain functional. In this context, functionality refers to the ability to direct packaging of the genome into the capsid shell and then allow for expression in the host cell to be infected or target cell. In the context of the present invention a capsid protein shell may be of a different serotype than the rAAV vector genome ITR.

A nucleic acid molecule represented by a nucleic acid sequence of choice is preferably inserted between the rAAV genome or ITR sequences as identified above, for example an expression construct comprising an expression regulatory element operably linked to a coding sequence and a 3' termination sequence. Said nucleic acid molecule may also be called a transgene.

"AAV helper functions" generally refers to the corresponding AAV functions required for rAAV replication and packaging supplied to the rAAV vector *in trans.* AAV helper functions complement the AAV functions which are missing in the rAAV vector, but they lack AAV ITRs (which are provided by the rAAV vector genome). AAV helper functions include the two major ORFs of AAV, namely the *rep* coding region and the *cap* coding region or functional substantially identical sequences thereof. Rep and Cap regions are well known in the art, see *e.g.* Chiorini et al. (1999, J. of Virology, Vol 73(2): 1309-1319) or US 5,139,941, incorporated herein by reference in its entirety. The AAV helper functions can be supplied on an AAV helper construct. Introduction of the helper construct into the host cell can occur e.g. by transformation, transfection, or transduction prior to or concurrently with the introduction of the rAAV genome present in the rAAV vector as identified herein. The AAV helper constructs of the invention may thus be chosen such that they produce the desired combination of serotypes for the rAAV vector's capsid protein shell on the one hand and for the rAAV genome present in said rAAV vector replication and packaging on the other hand.

"AAV helper virus" provides additional functions required for AAV replication and packaging. Suitable AAV helper viruses include adenoviruses, herpes simplex viruses (such as HSV types 1 and 2) and vaccinia viruses. The additional functions provided by the helper virus can also be introduced into the host cell via plasmids, as described in US 6,531,456 incorporated herein by reference in its entirety.

"Transduction" refers to the delivery of a nucleic acid molecule into a recipient host cell by a viral vector. For example, transduction of a target cell by an AAV vector according to the invention leads to transfer of the recombinant viral genome contained in that vector into the transduced cell.

### Expression

Expression may be assessed by any method known to a person of skill in the art. For example, expression may be assessed by measuring the levels of gene expression in the transduced tissue on the level of the mRNA or the protein by standard assays known to a person of skill in the art, such as qPCR, RNA sequencing, Northern blot analysis, Western blot analysis, mass spectrometry analysis of protein-derived peptides or ELISA.

Expression may be assessed at any time after administration of the recombinant viral genome, expression vector or composition as described herein. In some embodiments herein, expression may be assessed after 1 week, 2 weeks, 3 weeks, 4, weeks, 5 weeks, 6 weeks, 7 weeks, 8 weeks, 9, weeks, 10 weeks, 11 weeks, 12 weeks, 14 weeks, 16 weeks, 18 weeks, 20 weeks, 22 weeks, 24 weeks, 28 weeks, 32 weeks, 36 weeks, 40 weeks, or more.

### Codon optimization

"Codon optimization", as used herein, refers to the processes employed to modify an existing coding sequence, or to design a coding sequence, for example, to improve translation in an expression host cell or organism of a transcript RNA molecule transcribed from the coding sequence, or to improve transcription of a coding sequence. Codon optimization includes, but is not limited to, processes including selecting codons for the coding sequence to suit the codon preference of the expression host organism. For example, to suit the codon preference of mammalian, preferably of murine, canine or human expression hosts. Codon optimization also eliminates elements that potentially impact negatively RNA stability and/or translation (e. g. termination sequences, TATA boxes, splice sites, ribosomal entry sites, repetitive and/or GC rich sequences and RNA secondary structures or instability motifs). In some embodiments, codon-optimized sequences show at least 3%, 5%, 10%, 15%, 20%, 25%, 30%, 40%, 50%, 60%, 70%, 80%, 90%, 100% or more increase in gene expression, transcription, RNA stability and/or translation compared to the original, non-codon-optimized sequence.

### General terms

In this document and in its claims, the verb "to comprise" and its conjugations is used in its non-limiting sense to mean that items following the word are included, but items not specifically mentioned are not excluded. In addition, the verb "to consist" may be replaced by "to consist essentially of" meaning that a composition as described herein may comprise additional component(s) than the ones specifically identified, said additional component(s) not altering the unique characteristics of the invention. In addition, the verb "to consist" may be replaced by "to consist essentially of" meaning that a method or use as described herein may comprise additional step(s) than the ones specifically identified, said additional step(s) not altering the unique characteristic of the invention. In addition, the verb "to consist" may be replaced by "to consist essentially of" meaning that a nucleotide or amino acid sequence as described herein may comprise additional nucleotides or amino acids than the ones specifically identified, said additional nucleotides or amino acids not altering the unique characteristics of the invention.

Reference to an element by the indefinite article "a" or "an" does not exclude the possibility that more than one of the element is present, unless the context clearly requires that there be one and only one of the elements. The indefinite article "a" or "an" thus usually means "at least one".

As used herein, with "at least" a particular value means that particular value or more. For example, "at least 2" is understood to be the same as "2 or more" i.e., 2, 3, 4, 5, 6, 7, 8, 9, 10, 11, 12, 13, 14, 15, ..., etc.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The word "about" or "approximately" when used in association with a numerical value (e.g. about 10) preferably means that the value may be the given value (of 10) more or less 1% of the value.

As used herein, the term "and/or" indicates that one or more of the stated cases may occur, alone or in combination with at least one of the stated cases, up to with all of the stated cases.

Various embodiments are described herein. Each embodiment as identified herein may be combined together unless otherwise indicated.

All patent applications, patents, and printed publications cited herein are incorporated herein by reference in the entireties, except for any definitions, subject matter disclaimers or disavowals, and except to the extent that the incorporated material is inconsistent with the express disclosure herein, in which case the language in this disclosure controls.

One skilled in the art will recognize many methods and materials similar or equivalent to those described herein, which could be used in the practice of the present invention. Indeed, the present invention is in no way limited to the methods and materials described.

The present invention is further described by the following examples which should not be construed as limiting the scope of the invention.

### Description of the figures

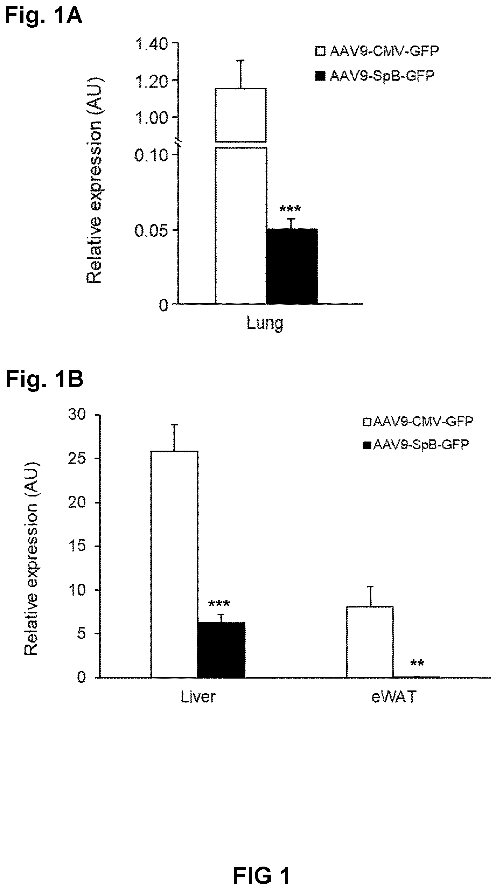

**Figure 1****. Comparison of lung-specific expression between AAV9-SpB-GFP and AAV9-CMV-GFP vectors.** Mice were treated with AAV9 vectors encoding the marker protein GFP under the control of the shortened SpB promoter (AAV9-SpB-GFP) or under the control of the ubiquitous CMV promoter (AAV9-CMV-GFP). A dose of 1.75x10¹² viral genomes (vg) of AAV9-SpB-GFP or AAV9-CMV-GFP vectors was administered intravenously (IV) via tail vein. GFP expression levels were evaluated by quantitative PCR four weeks post-AAV administration in lung (**Fig. 1A**), liver and epididymal white adipose tissue (eWAT) (**Fig. 1B**). Results are expressed as mean relative expression (AU) + SEM. **p<0.01, ***p<0.001 vs AAV9-CMV-GFP.

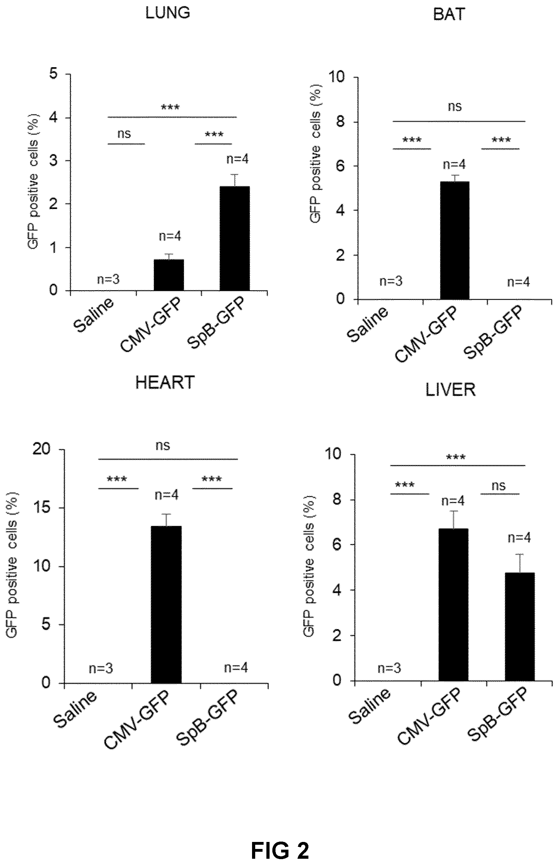

**Figure 2****. Quantification of GFP positive cells in tissues of mice treated with AAV9-SpB-GFP and AAV9-CMV-GFP vectors.** Mice were treated with AAV9 vectors encoding the marker protein GFP under the control of the shortened SpB promoter (AAV9-SpB-GFP) or under the control of the ubiquitous CMV promoter (AAV9-CMV-GFP). A dose of 1.75x10¹² viral genomes (vg) of AAV9-SpB-GFP or AAV9-CMV-GFP vectors was administered intravenously (IV) via tail vein. Saline solution was used as control. The percentage of GFP positive cells in lung, liver, heart, and brown adipose tissue (BAT) in mice treated with AAV9-SpB-GFP, AAV9-CMV-GFP, or saline was quantified. Results were evaluated by quantitative PCR four weeks post-AAV administration. Results are expressed as mean percentage + SEM. n= 3-4. ***p< 0.05, ns not significant. Straight lines connecting bars indicate the comparisons.

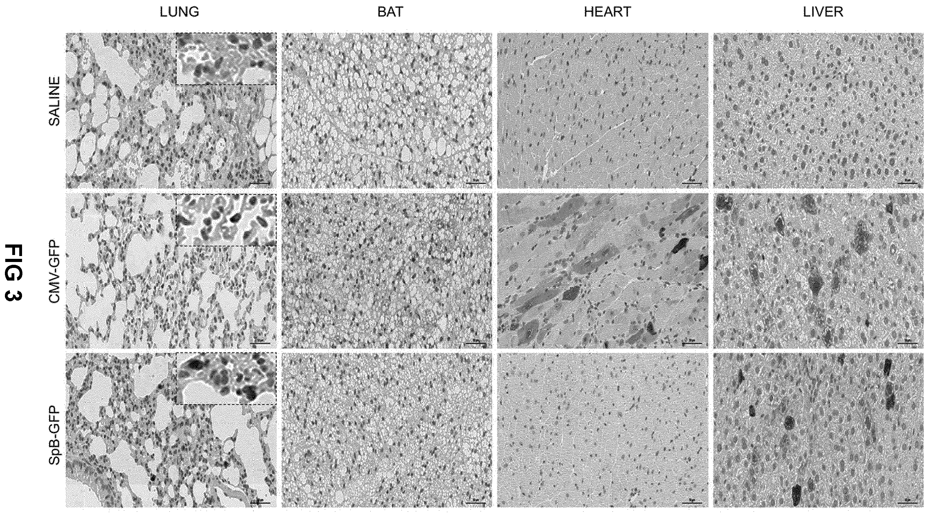

**Figure 3****. Visualisation of GFP positive cells in tissues of mice treated with AAV9-SpB-GFP and AAV9-CMV-GFP vectors.** Mice were treated with AAV9 vectors encoding the marker protein GFP under the control of the shortened SpB promoter (AAV9-SpB-GFP) or under the control of the ubiquitous CMV promoter (AAV9-CMV-GFP). A dose of 1.75x10¹² viral genomes (vg) of AAV9-SpB-GFP or AAV9-CMV-GFP vectors was administered intravenously (IV) via tail vein. Results were evaluated four weeks post-AAV administration. Saline solution was used as control. GFP positive cells in lung, brown adipose tissue (BAT), heart, and liver were detected by immunohistochemistry staining using a rabbit monoclonal antibody against GFP and visualised by light microscopy.

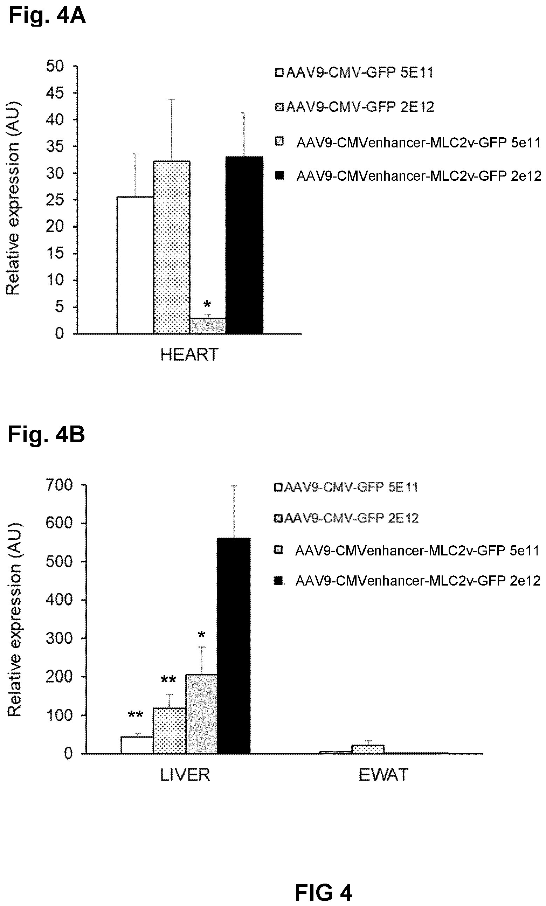

**Figure 4****. Comparison of heart-specific expression between AAV9-CMVenh-MLC2v-GFP and AAV9-CMV-GFP vectors.** Mice were treated with two different doses (5x10¹¹ or 2x10¹² viral genomes (vg)/mouse) of AAV9 vectors encoding the marker protein GFP under the control of the CMVenh-MLC2v promoter (AAV9-CMVenhancer-MLC2v-GFP) or under the control of the ubiquitous CMV promoter (AAV9-CMV-GFP), administered intravenously (IV) via tail vein. GFP expression levels were evaluated by quantitative PCR three weeks post-AAV administration in heart **(****Fig. 4A**), liver and epididymal white adipose tissue (eWAT) **(****Fig. 4B****).** Results are expressed as mean relative expression (AU) + SEM. *p<0.05, **p<0.01 vs AAV9-CMVenhancer-MLC2v-GFP 2x10¹²vg/mouse.

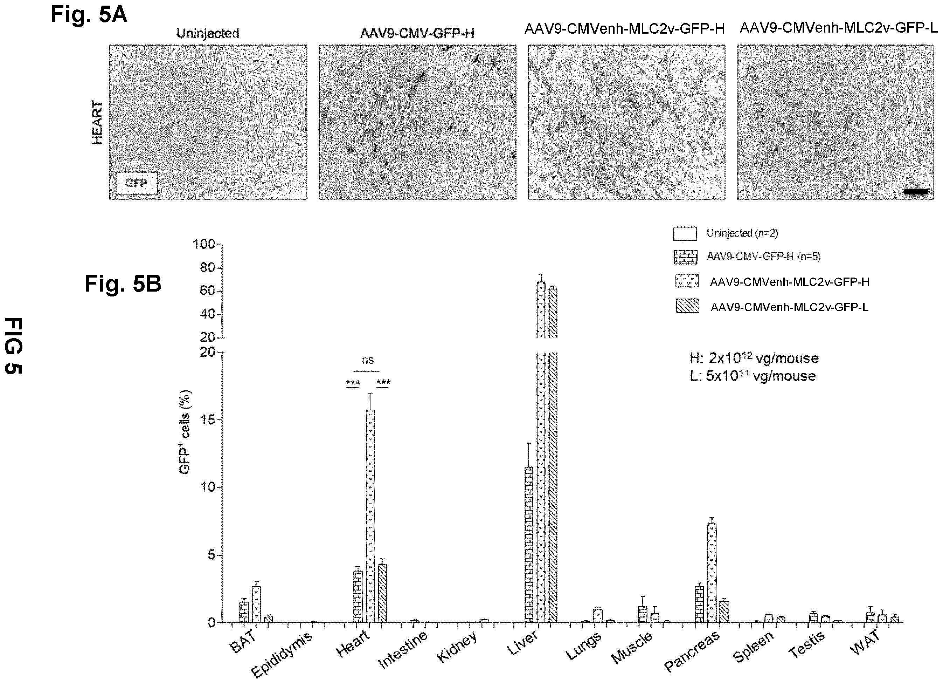

**Figure 5****. Visualisation and quantification of GFP positive cells in tissues of mice treated with AAV9-CMVenh-MLC2v-GFP and AAV9-CMV-GFP vectors.** Mice were treated with two different doses (5x10¹¹ (low (L) dose) or 2x10¹² (high (H) dose) viral genomes (vg)/mouse) of AAV9 vectors encoding the marker protein GFP under the control of the CMVenh-MLC2v promoter (AAV9-CMVenh-MLC2v-GFP) or under the control of the ubiquitous CMV promoter (AAV9-CMV-GFP), administered intravenously via tail vein. Results were evaluated three weeks post-AAV administration. Uninjected mice were used as control. GFP positive cells in heart, brown adipose tissue (BAT), epididymis, intestine, kidney, liver, lungs, muscle, pancreas, spleen, testis and white adipose tissue (WAT) were detected by immunohistochemistry staining using a rabbit monoclonal antibody against GFP and visualised by light microscopy. In **Fig. 5A** GFP positive cells in the heart are visualised . In **Fig. 5B** the percentage of GFP positive cells in brown adipose tissue (BAT), epididymis, heart, intestine, kidney, liver, lungs, muscle, pancreas, spleen, testis, and white adipose tissue (WAT) is quantified. Results are expressed as mean percentage + SEM. n= 2-6. ***p< 0.05, ns not significant. Straight lines connecting bars indicate the comparisons.

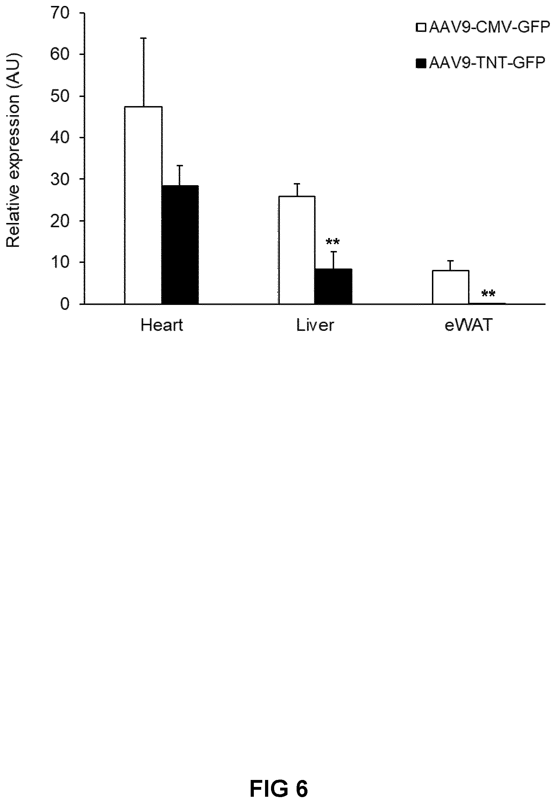

**Figure 6****. Comparison of heart-specific expression between AAV9-TNT-GFP and AAV9-CMV-GFP vectors.** 7-week-old male C57BI6 mice were treated with AAV9 vectors encoding the marker protein GFP under the control of the shortened TNT promoter (AAV9-TNT-GFP) or under the control of the ubiquitous CMV promoter (AAV9-CMV-GFP). A dose of 1.75x10¹² viral genomes (vg) of AAV9-TNT-GFP or AAV9-CMV-GFP vectors was administered intravenously (IV) via tail vein. GFP expression levels were evaluated by quantitative PCR four weeks post-AAV administration in heart, liver and epididymal white adipose tissue (eWAT). Results are expressed as mean relative expression (AU) + SEM. **p<0.01 vs AAV9-CMV-GFP.

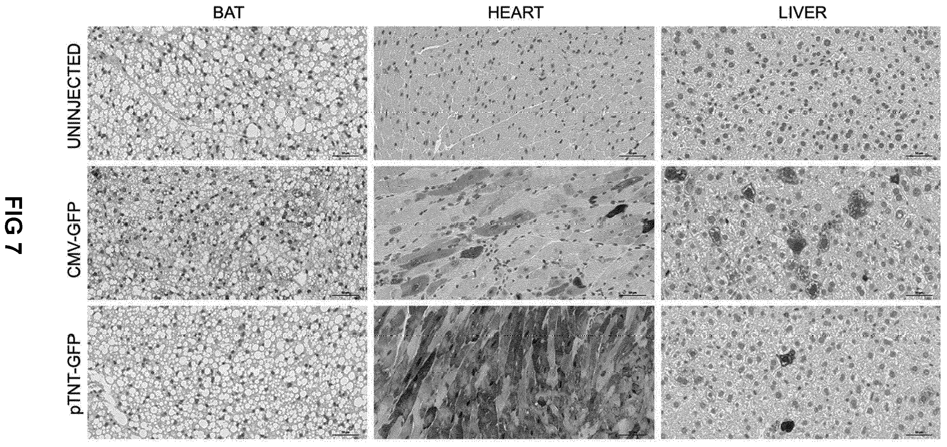

**Figure 7****. Visualisation of GFP positive cells in tissues of mice treated with AAV9-TNT-GFP and AAV9-CMV-GFP vectors.** 7-week-old male C57BI6 mice were treated with AAV9 vectors encoding the marker protein GFP under the control of the shortened TNT promoter (AAV9-TNT-GFP) or under the control of the ubiquitous CMV promoter (AAV9-CMV-GFP). A dose of 1.75x10¹² viral genomes (vg) of AAV9-TNT-GFP or AAV9-CMV-GFP vectors was administered intravenously (IV) via tail vein. Uninjected mice were used as control. Results were evaluated four weeks post-AAV administration. GFP positive cells in brown adipose tissue (BAT), heart, and liver were detected by immunohistochemistry staining using a rabbit monoclonal antibody against GFP and visualised by light microscopy.

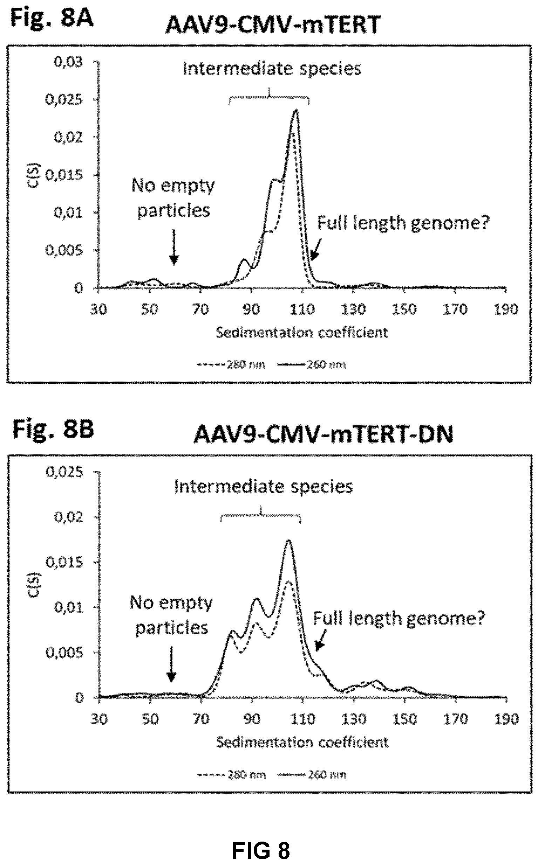

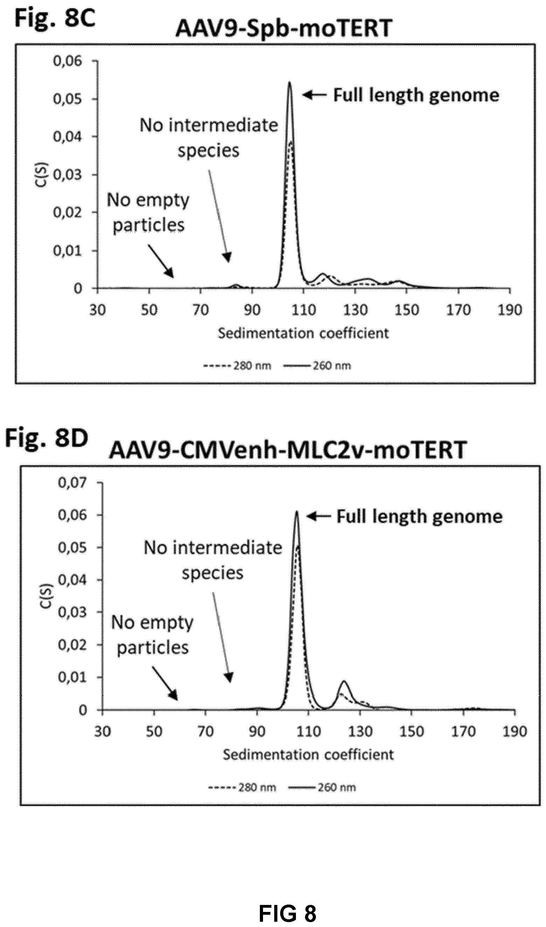

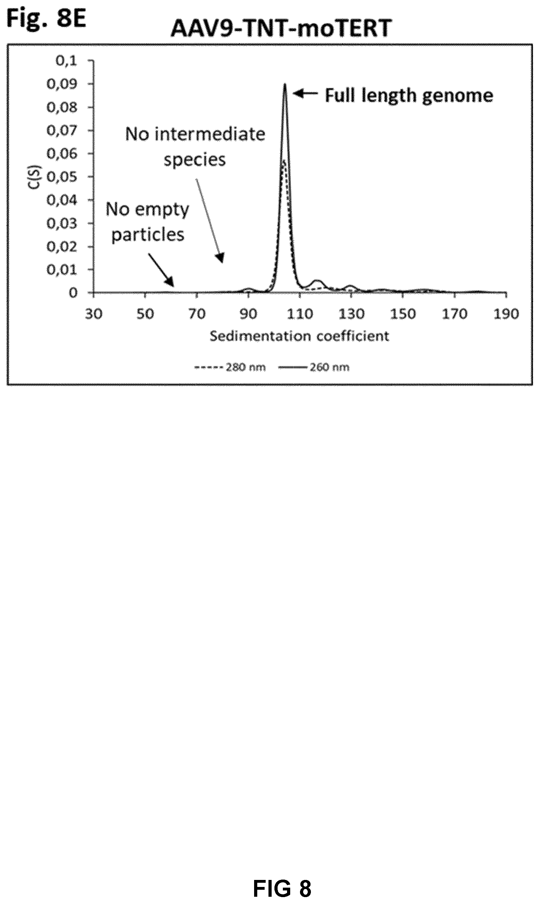

**Figure 8****. Characterization of AAV vectors by sedimentation velocity analytical ultracentrifugation.** AAV9-CMV-mTert (**Fig. 8A****,** 5291 nucleotides), AAV9-CMV-mTERT-DN (**Fig. 8B****,** 5291 nucleotides), AAV9-SpB-moTERT (**Fig. 8C****,** 4638 nucleotides), AAV9-CMVenh-MLC2v-moTERT (**Fig. 8D****,** 4676 nucleotides), AA9-TNT-moTERT (**Fig. 8E****,** 4536 nucleotides). Sedimentation velocity centrifugation was performed at 20,000 rpm. Particle sedimentation was recorded at 260 nm and 280 nm in the same run. The relative percentage of each peak in the C(S) distribution was calculated based on the molar concentration of each species in relation to the sum of the molar concentration of all species in the distribution. Peaks corresponding to empty vectors, intermediate species and vectors containing full length genomes are indicated with arrows.

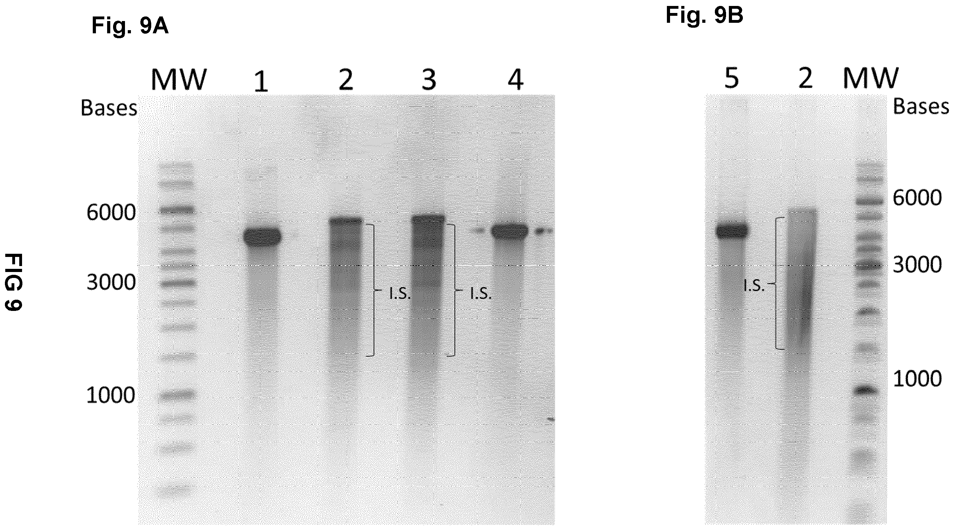

**Figure 9****. Characterization of AAV vectors by alkaline agarose gel electrophoresis.** Purified vector DNA was subjected to agarose gel electrophoresis under denaturing conditions. Band size and quality (presence of smear) were examined to assess genome integrity. MW denotes the DNA ladder. I.S. denotes the presence of intermediate species. **(****Fig. 9A****)** AAV9-SpB-moTERT (well "1", 4638 nucleotides), AAV9-CMV-mTert (well "2", 5291 nucleotides), AAV9-CMV-mTERT-DN (well "3", 5291 nucleotides), AAV9-CMVenh-MLC2v-moTERT (well "4", 4676 nucleotides). **(****Fig. 9B****)** AA9-TNT-moTERT (well "5", 4536 nucleotides), AAV9-CMV-mTert (well "2", 5291 nucleotides).

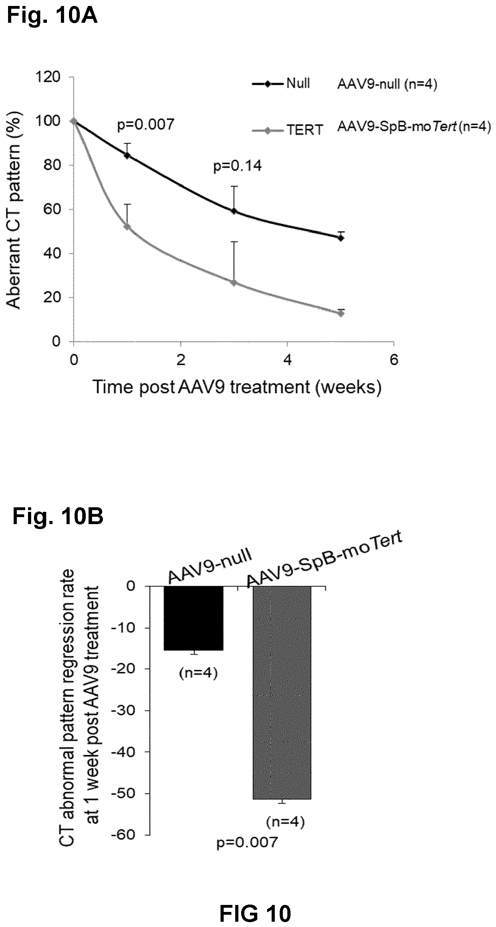

**Figure 10****. Evaluation of AAV9-SpB-moTERT therapeutic benefit in a pulmonary fibrosis mouse model.** Bleomycin (0.5 mg/kg body weight) was instilled intratracheally in 8-week-old G3-Tert-/- male mice. Two weeks post-Bleomycin insult, a computerized tomography (CT) scan was performed for each mouse to diagnose bona-fide pulmonary fibrotic pattern. Mice diagnosed with pulmonary fibrosis were intravenously (IV) administered either with 2x10¹² vg of AAV9-SpB-moTERT or AAV9-null vector (negative control). One, 3 and 5 weeks post-viral treatment a CT scan was performed for measurement of the decrease in the affected lung volume showing an aberrant CT pattern relative to the start of the treatment. **(****Fig. 10A**) Time series of decrease in aberrant fibrotic pattern (%) as measured by CT scan. **(****Fig. 10B****)** CT abnormal pattern regression rate at 1 week post-treatment. Results are expressed as mean percentage of initial fibrotic pattern + SEM. n = 4.

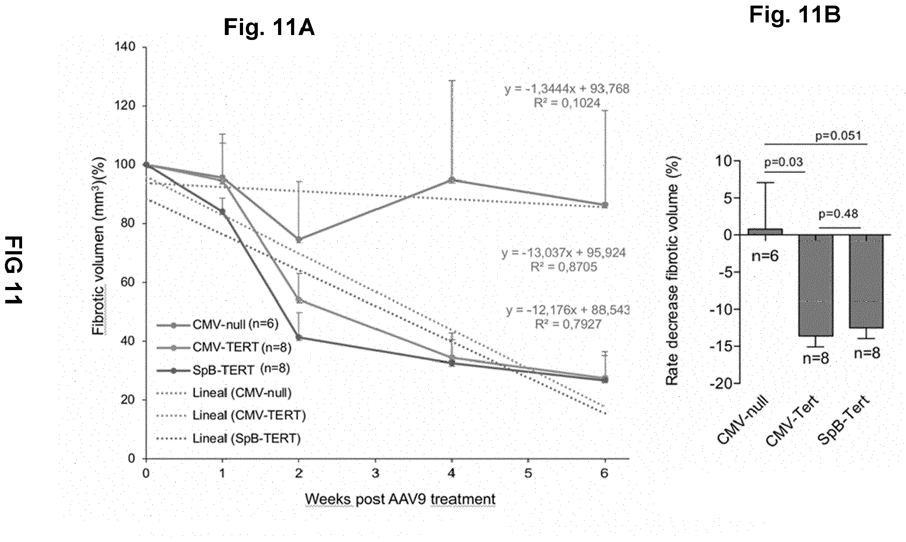

**Figure 11****. Evaluation of therapeutic efficacy of by AAV9-SpB-moTERT-mediated pulmonary fibrosis treatment in a mouse model.** Bleomycin (0.5-0.7 mg/kg body weight) was instilled intratracheally in 8-week-old G3-Tert-/- male mice. Mice diagnosed with pulmonary fibrosis were intravenously (IV) administered either with 2x10¹² vg of AAV-CMV-null, with 2x10¹² vg of AAV9-CMV-mTERT or with 2x10¹² vg of AAV9-SpB-moTERT. Disease progression was followed by weekly CT-scans. A weekly CT scan was performed for measurement of the decrease in the fibrotic lung volume relative to the start of the treatment. **(****Fig. 11A****)** Time series of decrease in aberrant fibrotic volume (%) as measured by CT scan. **(****Fig. 11B****)** Rate of decrease of affected volume calculated during the first six weeks of treatment.

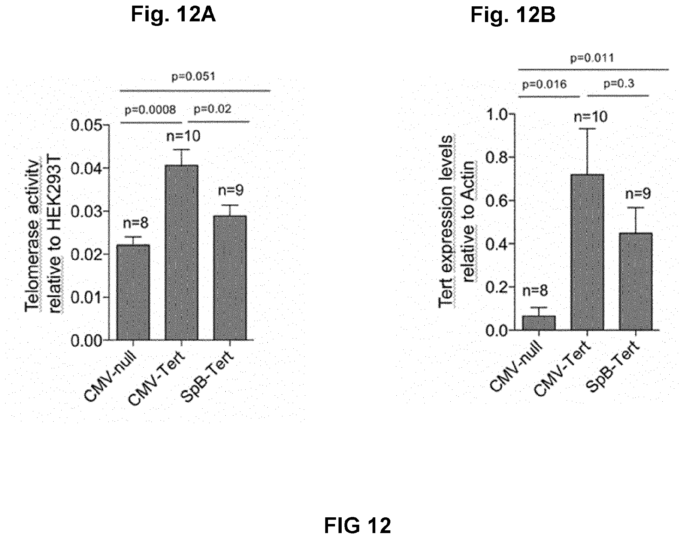

**Figure 12****. TERT expression levels and activity in lung samples of mice with pulmonary fibrosis treated with AAV9-SpB-moTERT.** Bleomycin (0.5-0.7 mg/kg body weight) was instilled intratracheally in 8-week-old G3-Tert-/- male mice. Mice diagnosed with pulmonary fibrosis were intravenously (IV) administered either with 2x10¹² vg of AAV-CMV-null, with 2x10¹² vg of AAV9-CMV-mTERT or with 2x10¹² vg of AAV9-SpB-moTERT. Mice were sacrificed at 8 weeks post-treatment and TERT expression levels and activity were measured in lung samples. **(****Fig. 12A****)** TERT activity (relative to HEK293T) were measured in lung samples. **(****Fig. 12B****)** TERT expression levels (relative to actin). Results are expressed as mean percentage + SEM. n=8-10.

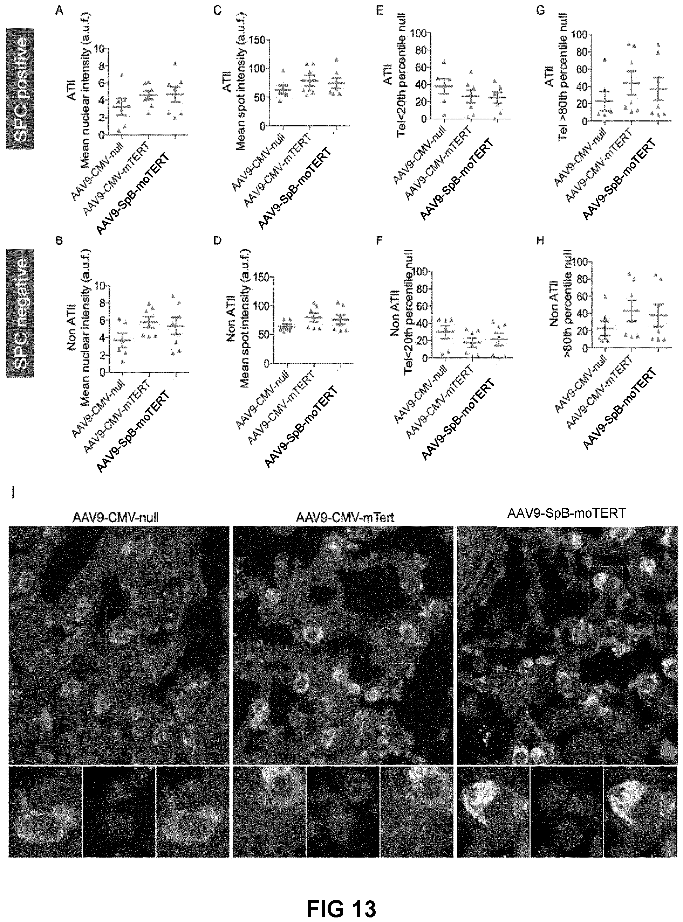

**Figure 13****. Telomere length analysis in lung samples of AAV9-CMV-null, AAV9-CMV-mTERT and AAV9-SpB-moTERT treated mice.** A-B. Mean nuclear intensity in ATII cells SPC positive (A) and SPC negative cells (B). C-D. Mean telomere intensity in ATII cells SPC positive (C) and SPC negative cells (D). E-F. Percentage of short telomeres below below 20th percentile of AAV9-CMV-null control samples in ATII cells SPC positive (E) and SPC negative cells (F). G-H. Percentage of short telomeres below below 20th percentile of AAV9-CMV-null control samples in ATII cells SPC positive (E) and SPC negative cells (F). G-H. Percentage of long telomeres above 80th percentile of AAV9-CMV-null control samples in ATII cells SPC positive (G) and SPC negative cells (H). I. Representative images of SPC immunofluorescence and telomeric Fish in lung samples.

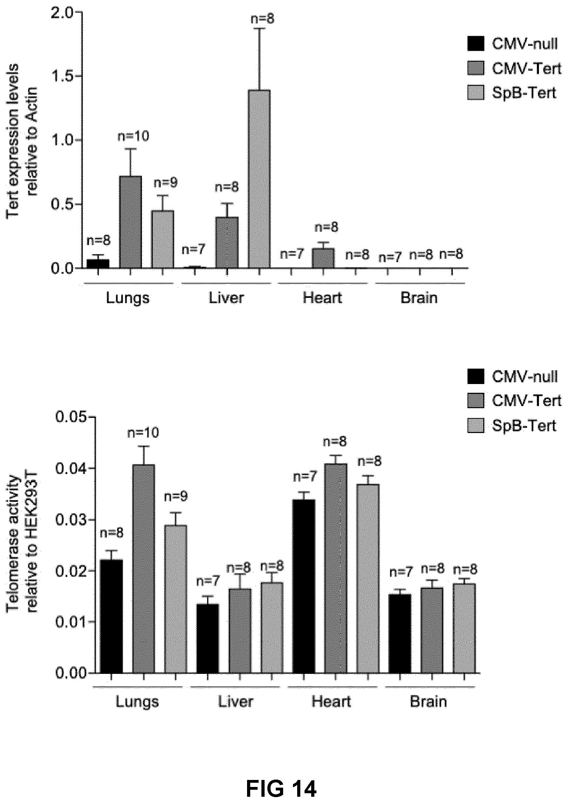

**Figure 14****. Quantification of Tert expression levels and telomerase activity (TRAP) in lung, liver, heart and brain samples.**

### Examples

### General procedures to the Examples

### Subject characteristics

For evaluation of tissue-specificity of shortened human SpB, CMVenh-MLC2v and TNT promoters, male C57BI/6J mice were used. Mice were fed *ad libitum* with a standard diet (2018S Teklad Global Diets^{®}, Harlan Labs., Inc., Madison, WI, US) and kept under a light-dark cycle of 12 h (lights on at 8:00 a.m.) and stable temperature (22°C ± 2). Prior to tissue sampling, mice were anesthetized by means of inhalational anesthetic isoflurane (IsoFlo^{®}, Abbott Laboratories, Abbott Park, IL, US) and decapitated. Tissues of interest were excised and kept at -80°C or with formalin until analysis. All experimental procedures were approved by the Ethics Committee for Animal and Human Experimentation of the Universitat Autònoma de Barcelona.

### Recombinant AAV vectors

Single-stranded AAV vectors of serotype 9 were produced by triple transfection of HEK293 cells according to standard methods (Ayuso, E. et al., 2010. Curr Gene Ther. 10(6):423-36). Cells were cultured in 10 roller bottles (850 cm², flat; Corning^{™}, Sigma-Aldrich Co., Saint Louis, MO, US) in DMEM 10% FBS to 80% confluence and co-transfected using the calcium phosphate method with a plasmid carrying the expression cassette flanked by the AAV2 ITRs, a helper plasmid carrying the AAV2 *rep* gene and the AAV of serotype 9 *cap* gene, and a plasmid carrying the adenovirus helper functions. Transgenes used were: mouse codon-optimized (SEQ ID NO: 3) TERT coding-sequence driven by 1) the shortened human SpB promoter (SEQ ID NO: 12); 2) the CMV enhancer fused to shortened human MLC2v promoter (SEQ ID NO: 15); or 3) the shortened human TNT promoter (SEQ ID NO: 16). Wild-type mouse TERT coding sequence including 3' UTR (SEQ ID NO: 1) driven by the ubiquitous CMV promoter was used as control (Bernardes de Jesus, B. et al., 2012. EMBO Mol Med 4(8):691-704). A noncoding plasmid was used to produce null vectors. AAV were purified with an optimized method based on a polyethylene glycol precipitation step and two consecutive cesium chloride (CsCI) gradients. This second-generation CsCI-based protocol reduced empty AAV capsids and DNA and protein impurities dramatically (Ayuso, E. et al., 2010. Curr Gene Ther. 10(6):423-36). Purified AAV vectors were dialyzed against PBS, filtered and stored at -80°C. Viral vectors were determined by fluorescence using the Quant-iT^{™} PicoGreen^{™} dsDNA Assay Kit (Invitrogen). A phage lambda DNA was used as standard curve to calculate the titer of viral vectors. The vectors were constructed according to molecular biology techniques well known in the art.

### Systemic administration of AAV vectors

The appropriate amount of the AAV solution was diluted in PBS with 0.001% Pluronic^{®} and was manually injected into the lateral tail vein without exerting pressure at the moment of delivery. Before the injection, the animals were put under a 250 W infrared heat lamp (Philips NV, Amsterdam, NL) for a few minutes to dilate the blood vessels and facilitate viewing and easier access to the tail vein. A plastic restrainer (Harvard Apparatus, Holliston, MA, US) was used to secure the animal for injection. No anesthesia was used since an appropriate restraining device was employed. A 30-gauge needle was utilized to inject the animals.

### RNA analysis

Total RNA was obtained from different tissues using isolation reagents (Tripure, Roche, for lung, liver and heart samples and QIAzol, Qiagen, for eWAT samples), and the RNeasy Tissue Minikit (Qiagen NV, Venlo, NL), according to the manufacturer's protocol. In order to eliminate residual viral genomes, total RNA was treated with DNAsel (Qiagen NV, Venlo, NL). The concentration and purity of the obtained RNA was determined using a Nanodrop (ND-1000, ThermoCientific). For RT-PCR, 1 µg of RNA samples was reverse-transcribed using the Transcriptor First Strand cDNA Synthesis Kit (04379012001, Roche, California, USA), according to the manufacturer's protocol. Real-time quantitative PCR was performed in a SmartCyclerll^{®} (Cepheid, Sunnyvale, USA) using LightCycler 480 SYBR Green I Master (Roche, ref. 04887352001, Roche Diagnostics, Germany). Data was normalized with RpIp0 values and analyzed as previously described (Pfaffl, M., Nucleic Acids Res. 2001; 29(9):e45).

### Characterization of AAV vectors by sedimentation velocity analytical ultracentrifugation

AAV vectors were characterized by analytical ultracentrifugation (AUC). Four hundred and fifty microliters of sample were loaded into the sample sector of a two-sector velocity cell, and 450 µl of PBS + 0.001% PF68 was loaded into the corresponding reference sector (Beckman-Coulter analytical ultracentrifuge Optima XLA equipped with UV-VIS absorbance optics). Sedimentation velocity centrifugation was performed at 20,000 rpm. Particle sedimentation was recorded at 260 nm and 280 nm in the same run. The relative percentage of each peak in the C(S) distribution was calculated based on the molar concentration of each species in relation to the sum of the molar concentration of all species in the distribution as previously described (Burnham, B. et al., 2015, Hum Gene Ther Methods 26(6):228-42).

### Determination of AAV vector genome integrity by alkaline agarose gel electrophoresis

AAV vector genome integrity was assessed by alkaline agarose gel electrophoresis (denaturing conditions) where single stranded DNA can be analyzed. First, 8 µg of vector genome was purified with the DNeasy blood and tissue kit (Qiagen), according to the manufacturer's protocol. Extracted DNA (∼1 µg) was mixed with alkaline loading buffer and boiled to 95 °C for 5 min and incubated on ice for 3 min. Next, DNA was subjected to agarose gel electrophoresis under denaturing conditions. Finally, band size and quality were examined to assess genome integrity.

### Visualisation of expression

GFP positive cells in heart, brown adipose tissue (BAT), epididymis, intestine, kidney, liver, lungs, muscle, pancreas, spleen, testis and white adipose tissue (WAT) were detected by immunohistochemistry staining using a rabbit monoclonal antibody against GFP (D5.1, Cell Signaling, #2956) and visualised by light microscopy.

### Computerized tomography

In-vivo lung imaging by computerized tomography (CT) was made on a high-resolution CT system (CT Locus, GE Healthcare) specifically designed for small laboratory animals. Mice were anesthetized with a 4% rate of isoflurane (IsoVet Braun) during the induction and 2% during the maintenance period (scanning time). Micro-CT image acquisition consisted of 400 projections collected in one full rotation of the gantry in approximately 14 min in a single bed focused on the legs, with a 450 µA/80kV X-ray tube. 2-D and 3-D images were obtained and analysed using the software program MicroView (GE Healthcare).

### Statistical analysis

All results are expressed as mean ± SEM. Differences between groups were compared by Student's t-test. Statistical significance was considered if P< 0.05.

### Example 1. AAV-mediated lung-specific transqene expression using a short version of the human SpB promoter

The long length of TERT sequences typically used in the art in conjunction with the low encapsidation capacity of AAV vectors (approximately 4.7 kb) highly restrict the number of promoter sequences that can be used in the expression cassette encoding telomerase without exceeding the encapsidation capacity of AAV vectors. To not exceed the AAV encapsidation capacity caused by utilization of lung-specific promoters in conjunction with TERT coding sequences and further components of the expression cassettes (i.e. polyA sequence, AAV ITRs) a shortened version (632-nucleotides, SEQ ID NO: 12) of the human surfactant protein B (SpB) promoter (size of the wild-type human SpB promoter: 1597-nucleotides, SEQ ID NO: 11) was designed. The wild-type human SpB promoter drives expression in Clara and ATII cells in the lung.

To study whether the shortened human SpB promoter was be able to mediate lung-specific transgene expression, AAV9 vectors encoding the marker protein GFP under the control of this shortened promoter (AAV9-SpB-GFP) or under the control of the ubiquitous CMV promoter (AAV9-CMV-GFP) were cloned and produced by triple transfection in HEK293 producer cells. A dose of 1.75x10¹² viral genomes (vg) of AAV9-SpB-GFP or AAV9-CMV-GFP vectors was administered intravenously (IV) to mice via tail vein. A group of mice administered IV with saline solution was used as control. Four weeks post-AAV administration, GFP expression levels were evaluated in lungs, liver and epididymal white adipose tissue (eWAT). Although GFP expression levels in lungs were lower than those mediated by the CMV promoter (Figure 1A), the shortened version of the SpB promoter prevented GFP expression in white adipose tissue and mediated 80% reduction of GFP expression levels in the liver (Figure 1B). Quantification of the percentage of GFP⁺ cells in lungs, liver, heart and brown adipose tissue (BAT), further confirmed the lung-specificity of the shortened SpB promoter (Figures 2 and 3). The use of shortened promoters could result in loss of specificity and promiscuous transgene expression in off-target tissues other than the lungs. Surprisingly, the shortened promoter used herein was able to mediate highly-specific lung expression.

### Example 2. AAV-mediated heart-specific transgene expression using shortened heart-specific promoters

A shortened (296-nucleotides, SEQ ID NO: 13) version of the cardiomyocyte-specific MLC2v promoter was also designed. In order to increase the promoter strength of this shortened version of the wild type MLC2v promoter, the CMV enhancer (380-nucleotides, SEQ ID NO: 14) was used in conjunction with said promoter (CMVenh-MLC2v, SEQ ID NO: 15).

To study whether the CMVenh-MLC2v promoter was capable of mediating specific expression in the heart, 8-week-old male C57BI6 mice were treated with two different doses (5x10¹¹ (low (L) dose) or 2x10¹² (high (H) dose) viral genomes (vg)/mouse) of AAV9-CMVenh-MLC2v-GFP vectors. As controls, two additional groups of mice were treated with the same doses of AAV9 vectors encoding GFP under the control of the ubiquitous CMV promoter (AAV9-CMV-GFP). Three weeks post-AAV, the animals were sacrificed and the levels of GFP expression and production were evaluated in different tissues. Mice treated with AAV9-CMVenh-MLC2v-GFP vectors, particularly at dose 2x10¹² vg/mouse, showed robust GFP expression in the heart (Figure 4A). In addition, the quantification of the percentage of GFP⁺ cells revealed higher number of transduced cells in heart in animals treated with the low and high dose of AAV9-CMVenh-MLC2v-GFP vectors than in mice administered with the high dose of AAV9-CMV-GFP vectors (Figure 5A and 5B). Moreover, the CMV-enhMLC2v promoter precluded GFP expression and production in testis, epididymis, white adipose tissue, skeletal muscle, lung, intestine, brain, spleen and kidney (Figures 4B and 5B). However, it was unable to restrict GFP production in pancreas, brown adipose tissue, or liver, tissues in which the percentage of GFP⁺ cells was similar in mice treated with AAV9-CMVenh-MLC2v-GFP or AAV9-CMV-GFP vectors (Figure 4B and 5B). Although transgene expression mediated by the CMVenh-MLC2v promoter was not completely heart-specific, the use of this promoter prevented GFP expression in a greater number of tissues in comparison with the CMV promoter.

To attain higher cardiac specificity than the one displayed by the CMVenh-MLC2v promoter, a shortened (544-nucleotides, SEQ ID NO: 16) version of the human troponin (TNT) promoter was used. To evaluate the strength and tissue-specificity of the shortened version of the TNT promoter, 1.75x10¹² vg of AAV9-TNT-GFP vectors were administered to 7-week-old male C57BI6 mice. As control, a group of mice was treated with 1.75x10¹² vg of AAV9-CMV-GFP vectors. Four weeks after AAV administration, mice were sacrificed and GFP expression levels were assessed. The shortened version of the TNT promoter mediated GFP expression levels in the heart similar to those obtained with the CMV promoter (Figure 6). Furthermore, mice treated with AAV9-TNT-GFP vectors showed lower levels of GFP in the liver than animals that received AAV9-CMV-GFP vectors (25% decrease) and absence of transgene expression in white and brown adipose tissue (Figures 6 and 7).

### Example 3. Optimization of the viral genome size prevents production of AAV intermediate species

Telomerase expression in lungs using AAV9 vectors encoding mouse telomerase under the control of the CMV promoter (AAV9-CMV-mTert) has been reported to mediate therapeutic benefit in pulmonary fibrosis and myocardial infarction (Povedano, J.M. et al. (2018) Elife.7: e31299; Bär, C. et al. (2014) Nat Comm Dec 18;5:5863). However, the size of the viral genome of AAV9-CMV-mTert vectors was 5291 nucleotides, which largely exceeded the AAV encapsidation limit of approximately 4.7 kb. Moreover, due to the large size of the viral genome, a polyA sequence could not be included in the expression cassette. Production of said AAV vectors resulted in vectors comprising several different intermediate species (Figure 8A). Noticeably, AAV vectors containing full length viral genomes were not clearly detected (Figure 8A). Moreover, analysis of genome integrity of AAV9-CMV-mTert vectors revealed a band of approximately 5.3 kb that corresponded to the full length AAV genome and a broad smear corresponding to truncated genomes (Figure 9A and 9B). Similar results were obtained when genome integrity and production of AAV intermediate species were evaluated for AAV9 vectors encoding a catalytically inactive form of murine telomerase under the control of the CMV promoter (AAV9-CMV-mTERT-DN) (Bernardes de Jesus, B. et al., 2012. EMBO Mol Med 4(8):691-704), whose viral genome size was also 5291 nucleotides (Figures 8B and 9A).

To avoid production of intermediate species in AAV vectors encoding telomerase under the control of the short version of the human SpB promoter, an exhaustive study of the coding sequences of mouse (mTERT) and human telomerases (hTERT) was carried out. The cDNA of mTERT (3786 nucleotides, SEQ ID NO: 1) was modified without altering the amino acid sequence of the protein by deleting the 3' UTR of the mRNA, resulting in a coding sequence of 3369 nucleotides in length (SEQ ID NO: 2), in order to minimize the length of the TERT encoding sequence. Moreover, said sequence was codon-optimized to improve protein production (moTERT, SEQ ID NO: 3). As a result of the decreased size of the TERT coding sequence and the use of the shortened human SpB promoter, the CMVenh-MLC2v promoter or the TNT promoter, the bovine growth hormone polyA sequence (SEQ ID NO: 18) could be included in the expression cassette without exceeding the encapsidation limit of AAV. Specifically, viral genomes size were: 4638 nucleotides for AAV9-SpB-moTERT (SEQ ID NO: 21), 4676 nucleotides for AAV9-CMVenh-MLC2v-moTERT (SEQ ID NO: 22) and 4536 nucleotides for AAV9-TNT-moTERT (SEQ ID NO: 23). Inclusion of a polyA sequence in the expression cassette can allow for improved TERT protein production due to increased mRNA stability, translational efficiency and for promotion of proper termination of mRNA avoiding the generation of unwanted 3' UTR sequences. No AAV intermediate species were detected for these vectors (Figures 8C-E). Genome integrity analysis revealed a band of approximately 4.7 kb that corresponded to full-length AAV genome and further confirmed no presence of truncated genomes (Figure 9A and 9B). Moreover, the higher intensity of the band corresponding to full-length vector genomes observed in AAV9-Spb-moTERT, AAV9-CMVenh-MLC2v-moTERT and AAV9-TNT-moTert indicated higher number of full-length genomes in these AAV preparations in comparison with AAV9-CMV-mTERT and AAV9-CMV-mTERT-DN (Figure 9A and 9B).

### Example 4. AAV9-SpB-moTERT vectors mediate therapeutic benefit in pulmonary fibrosis

Next, we evaluated whether AAV9-SpB-moTERT vectors could mediate therapeutic benefit in pulmonary fibrosis. A mouse model of pulmonary fibrosis induced by short telomeres was used (Povedano el al (2015) Cell Rep 12, 286-299). This model is based on the intratracheal instillation of a low dose of bleomycin (0.5 mg/kg body weight) in 8-week-old G3-*Tert*^{-/-} male mice, a dose that does not induce fibrosis in wild type mice with normal length telomeres. Two weeks post-Bleo insult, a computerized tomography (CT) scan was performed for each mouse to diagnose *bona-fide* pulmonary fibrotic pattern. Mice diagnosed with pulmonary fibrosis were intravenously (IV) administered either with 2x10¹² vg of AAV9-SpB-moTERT or AAV9-null vectors. One, 3 and 5 weeks post-viral treatment a CT scan was performed for longitudinal follow-up of the disease progression. The decrease in the lung affected volume showing an aberrant CT pattern relatively to the start of the treatment was significantly higher in mice transduced with AAV9-SpB-moTERT as compared to those transduced with AAV9-null already after one week (Figure 10A-B).

### Example 5. Comparative study of AAV9-CMV-mTERT and AAV9-SpB-moTERT efficacy in pulmonary fibrosis regression

The therapeutic efficacy in pulmonary fibrosis treatment mediated by AAV9-SpB-moTERT in comparison with AAV9-CMV-mTERT vectors was also evaluated. G3-Tert-/- male mice were administered intratracheally with bleomycin and two weeks post-bleo insult a CT was performed for each mouse to diagnose *bona-fide* pulmonary fibrotic pattern. Mice diagnosed with pulmonary fibrosis were IV administered either with 2x10¹² vg of AAV-CMV-null, with 2x10¹² vg of AAV9-CMV-mTERT or with 2x10¹² vg of AAV9-SpB-moTERT. Before bleomycin insult, an initial CT was performed for each mouse to calculate the volume of healthy lungs at baseline for further calculation of disease progression.

The disease progression was longitudinally followed up by CT at 1, 2, 4, 6 and 8 weeks post-AAV9 treatment. No changes in the affected volume were detected between week 6 and 8 post-AAV9 treatment, indicating chronification of the disease (Figure 11A). The rate of decrease of affected volume was therefore calculated during the first six weeks of treatment. The rate of decrease of the affected lung volume was similar in both treatments, AAV9-CMV-mTERT and AAV9-SpB-moTERT and faster than in the control group (Figures 11A and 11B). Mice were sacrificed at 8 weeks post-treatment. TERT expression levels and activity (TRAP) were measured in lung samples. Telomerase expression was analyzed by qPCR. Telomerase activity was measured as previously described (Herbert et al. Nat Protoc 1;583-1590, (2006), incorporated herein by reference in its entirety). The results demonstrate higher TERT expression and higher telomerase activity in both AAV9-CMV-mTERT and AAV9-SpB-moTERT-treated animals as compared to untreated ones (Figure 12A and 12B).

### Example 6. Telomere length, Tert expression and telomerase activity

We performed immune-q-Fish with anti-SPC, a specific antibody of ATII cells, and a telomeric probe for quantification of telomere intensity in lung sections of AAV9-CMV-mTert (n=7), AAV9-SpB-moTert (n=7) and AAV9-CMV-null (n=6) treated mice. Both AAV9-CMV-mTert and AAV9-SpB-moTert groups showed an increased mean nuclear and mean spot intensity in ATII cells as well as in SPC negative cells as compared to AAV9-CMV-null treated group (Fig 13 A-D). In addition, the percentage of short telomeres (below 20th percentile of AAV9-CMV-null control samples) was lower in AAV9-CMV-mTert and AAV9-SpB-moTert as compared to AAV9-CMV-null control samples (Fig. 13 E-F). The percentage of long telomeres (above 80th percentile of AAV9-CMV-null control samples) was higher in AAV9-CMV-mTert and AAV9-SpB-moTert as compared to AAV9-CMV-null control samples (Fig. 13 G-H). Representative images of the assay are shown in Fig. 13I. These results indicate a bona fide telomere length increase in AAV9-CMV-mTERT and in AAV9-SpB-moTERT treated lungs.

Expression levels of Tert and Telomerase activity have been determined in lungs, liver, brain and heart by q-PCR and TRAP analysis, respectively (Fig. 14). The results demonstrate that AAV9-SpB-moTERT is not expressed neither in heart nor in brain.

### Sequences

| **SEQ ID NO** | **Name** | **Description** | **Sequence type** |
|---|---|---|---|
| 1 | mTERT CDS + native 3' UTR | Mouse TERT CDS with native 3' UTR | nucleotide |
| 2 | mTERT CDS | Mouse TERT CDS | nucleotide |
| 3 | moTERT | Mouse TERT CDS codon optimized | nucleotide |
| 4 | mTERT protein | Mouse TERT protein | amino acid |
| 5 | hTERT long isoform | Human TERT long isoform CDS | nucleotide |
| 6 | hoTERT long isoform | Human TERT long isoform CDS codon optimized | nucleotide |
| 7 | hTERT long isoform protein | Human TERT long isoform protein | amino acid |
| 8 | hTERT short isoform | Human TERT short isoform CDS | nucleotide |
| 9 | hoTERT short isoform | Human TERT short isoform CDS codon optimized | nucleotide |
| 10 | hTERT short isoform protein | Human TERT short isoform protein | amino acid |
| 11 | SpB promoter full length | Full length version of surfactant protein B promoter | nucleotide |
| 12 | SpB promoter shortened | Shortened version of surfactant protein B promoter | nucleotide |
| 13 | MLC2v promoter shortened | Shortened version of ventricular myosin regulatory light chain 2 promoter | nucleotide |
| 14 | CMV enhancer | cytomegalovirus enhancer | nucleotide |
| 15 | CMVenh-MLC2v promoter | Fusion of CMV enhancer and shortened version of MLC2v promoter | nucleotide |
| 16 | TNT promoter shortened | Shortened version of human troponin promoter | nucleotide |
| 17 | kozak sequence | kozak sequence | nucleotide |
| 18 | BGH polyA | Bovine growth hormone polyA sequence | nucleotide |
| 19 | 5' ITR AAV2 | 5' ITR of AAV2 | nucleotide |
| 20 | 3' ITR AAV2 | 3' ITR of AAV2 | nucleotide |
| 21 | AAV9-SpB-moTERT genome | Lung expression vector viral genome | nucleotide |
| 22 | AAV9-CMVenh-MLC2v-moTERT genome | Heart expression vector viral genome | nucleotide |
| 23 | AAV9-TNT-moTert genome | Heart expression vector viral genome | nucleotide |

SEQ ID NO: 12 (SpB promoter shortened)

ATAGGGCTGTCTGGGAGCCACTCCAGGGCCACAGAAATCTTGTCTCTGACTCAGGGTATTTTGTTTTCTGTTTTGTGTAAATGCTCTTCTGACT

AATGCAAACCATGTGTCCATAGAACCAGAAGATTTTTCCAGGGGAAAAGGTAAGGAGGTGGTGAGAGTGTCCTGGGTCTGCCCTTCCAGGGC

TTGCCCTGGGTTAAGAGCCAGGCAGGAAGCTCTCAAGAGCATTGCTCAAGAGTAGAGGGGGCCTGGGAGGCCCAGGGAGGGGATGGGAGG

GGAACACCCAGGCTGCCCCCAACCAGATGCCCTCCACCCTCCTCAACCTCCCTCCCACGGCCTGGAGAGGTGGGACCAGGTATGGAGGCTT

GAGAGCCCCTGGTTGGAGGAAGCCACAAGTCCAGGAACATGGGAGTCTGGGCAGGGGGCAAAGGAGGCAGGAACAGGCCATCAGCCAGG

ACAGGTGGTAAGGCAGGCAGGAGTGTTCCTGCTGGGAAAAGGTGGGATCAAGCACCTGGAGGGCTCTTCAGAGCAAAGACAAACACTGAGG
TCGCTGCCACTCCTACAGAGCCCCCACGCCCCGCCCAGCTATAAGGGGCCATGCACCAAGCAGGGTACCCAGGCTGCAGAGGTGC

SEQ ID NO: 15 (CMVenh-MLC2v promoter)

GACATTGATTATTGACTAGTTATTAATAGTAATCAATTACGGGGTCATTAGTTCATAGCCCATATATGGAGTTCCGCGTTACATAACTTACGGTA

AATGGCCCGCCTGGCTGACCGCCCAACGACCCCCGCCCATTGACGTCAATAATGACGTATGTTCCCATAGTAACGCCAATAGGGACTTTCCA

TTGACGTCAATGGGTGGACTATTTACGGTAAACTGCCCACTTGGCAGTACATCAAGTGTATCATATGCCAAGTACGCCCCCTATTGACGTCAA

TGACGGTAAATGGCCCGCCTGGCATTATGCCCAGTACATGACCTTATGGGACTTTCCTACTTGGCAGTACATCTACGTATTAGTCATCGCTATT

ACCATGGTTAACGTTTAAACTTAGACAATGGCAGGACCCAGAGCACAGAGCATCGTTCCCAGGCCAGGCCCCAGCCACTGTCTCTTTAACCTT

GAAGGCATTTTTGGGTCTCACGTGTCCACCCAGGCGGGTGTCGGACTTTGAACGGCTCTTACTTCAGAAGAACGGCATGGGGTGGGGGGGC

TTAGGTGGCCTCTGCCTCACCTACAACTGCCAAAAGTGGTCATGGGGTTATTTTTAACCCCAGGGAAGAGGTATTTATTGTTCCACAGCAGGG

GCCGGCCAGCAGGCTCCTTGAATTC

SEQ ID NO: 16 (TNT promoter shortened)

CTCAGTCCATTAGGAGCCAGTAGCCTGGAAGATGTCTTTACCCCCAGCATCAGTTCAAGTGGAGCAGCACATAACTCTTGCCCTCTGCCTTCC

AAGATTCTGGTGCTGAGACTTATGGAGTGTCTTGGAGGTTGCCTTCTGCCCCCCAACCCTGCTCCCAGCTGGCCCTCCCAGGCCTGGGTTGC

TGGCCTCTGCTTTATCAGGATTCTCAAGAGGGACAGCTGGTTTATGTTGCATGACTGTTCCCTGCATATCTGCTCTGGTTTTAAATAGCTTATC

TGAGCAGCTGGAGGACCACATGGGCTTATATGGCGTGGGGTACATGTTCCTGTAGCCTTGTCCCTGGCACCTGCCAAAATAGCAGCCAACAC

CCCCCACCCCCACCGCCATCCCCCTGCCCCACCCGTCCCCTGTCGCACATTCCTCCCTCCGCAGGGCTGGCTCACCAGGCCCCAGCCCACA

TGCCTGCTTAAAGCCCTCTCCATCCTCTGCCTCACCCAGTCCCCGCTGAGACTGAGCAGACGCCTCCAGGATCTGTCGGCAG

SEQ ID NO: 21 (AAV9-SpB-moTERT genome)

CCTGCAGGCAGCTGCGCGCTCGCTCGCTCACTGAGGCCGCCCGGGCAAAGCCCGGGCGTCGGGCGACCTTTGGTCGCCCGGCCTCAGTG
AGCGAGCGAGCGCGCAGAGAGGGAGTGGCCAACTCCATCACTAGGGGTTCCTGCGGCCGCGATATCATAGGGCTGTCTGGGAGCCACTCC
AGGGCCACAGAAATCTTGTCTCTGACTCAGGGTATTTTGTTTTCTGTTTTGTGTAAATGCTCTTCTGACTAATGCAAACCATGTGTCCATAGAAC
CAGAAGATTTTTCCAGGGGAAAAGGTAAGGAGGTGGTGAGAGTGTCCTGGGTCTGCCCTTCCAGGGCTTGCCCTGGGTTAAGAGCCAGGCA
GGAAGCTCTCAAGAGCATTGCTCAAGAGTAGAGGGGGCCTGGGAGGCCCAGGGAGGGGATGGAGGGGAACACCCAGGCTGCCCCCAAC
CAGATGCCCTCCACCCTCCTCAACCTCCCTCCCACGGCCTGGAGAGGTGGGACCAGGTATGGAGGCTTGAGAGCCCCTGGTTGGAGGAAG
CCACAAGTCCAGGAACATGGGAGTCTGGGCAGGGGGCAAAGGAGGCAGGAACAGGCCATCAGCCAGGACAGGTGGTAAGGCAGGCAGGA
GTGTTCCTGCTGGGAAAAGGTGGGATCAAGCACCTGGAGGGCTCTTCAGAGCAAAGACAAACACTGAGGTCGCTGCCACTCCTACAGAGCC
CCCACGCCCCGCCCAGCTATAAGGGGCCATGCACCAAGCAGGGTACCCAGGCTGCAGAGGTGCGAATTCATTTAAATTCTAGATCGCTAGCA
CGCGTGCCACCATGACAAGAGCCCCTAGATGTCCTGCCGTCAGAAGCCTGCTGAGAAGCAGATACAGAGAAGTGTGGCCCCTGGCCACCTT
CGTCAGAAGGCTTGGACCTGAAGGCAGACGGCTGGTTCAACCTGGCGACCCCAAGATCTACAGAACCCTGGTGGCTCAGTGCCTCGTGTGT
ATGCACTGGGGATCTCAGCCACCTCCTGCCGATCTGAGCTTTCACCAGGTGTCCAGCCTGAAAGAACTGGTGGCCAGAGTGGTGCAGAGAC
TGTGCGAGAGAAACGAGAGGAACGTGCTGGCCTTCGGCTTCGAGCTGCTGAATGAAGCTAGAGGCGGCCCTCCTATGGCCTTCACATCTAG
CGTGCGGAGCTACCTGCCTAACACCGTGATCGAGACACTGAGAGTGTCCGGCGCTTGGATGCTGCTGCTGAGCAGAGTGGGAGATGACCTG
CTGGTGTACCTGCTGGCTCACTGTGCCCTCTATCTGCTGGTGCCTCCTAGCTGCGCTTACCAAGTGTGCGGAAGCCCTCTGTACCAGATCTG
TGCCACCACCGACATCTGGCCTAGCGTGTCCGCTTCCTACAGACCCACAAGACCCGTGGGCAGAAACTTTACCAACCTGAGATTCCTGCAGC
AGATCAAGAGCAGCTCCAGGCAAGAGGCCCCTAAGCCTCTTGCTCTGCCTAGCAGAGGCACCAAGAGACACCTGAGCCTGACCAGCACAAG
CGTGCCCTCTGCCAAGAAAGCCAGATGCTACCCCGTGCCTAGAGTGGAAGAGGGCCCACATAGACAGGTGCTGCCTACACCTAGCGGCAAG
AGCTGGGTTCCATCTCCTGCTAGAAGCCCTGAGGTGCCAACAGCCGAGAAGGACCTGAGCAGCAAGGGCAAAGTGTCCGACCTGTCTCTGA
GCGGCAGCGTGTGTTGCAAGCACAAGCCTAGCAGCACCAGCCTGCTGTCCCCACCTAGACAGAACGCTTTCCAGCTGAGGCCCTTTATCGA
GACAAGACACTTCCTGTACAGCAGAGGCGACGGCCAAGAGAGACTGAACCCTAGCTTCCTGCTGTCTAACCTGCAGCCTAACCTGACCGGC
GCTAGAAGGCTGGTGGAAATCATCTTCCTGGGCAGCAGACCCAGAACCAGCGGACCTCTGTGTAGAACCCACAGACTGAGCAGACGGTACT
GGCAGATGAGGCCCCTGTTCCAGCAGCTCCTGGTCAACCATGCCGAGTGTCAGTATGTGCGGCTGCTGAGGTCCCACTGCAGATTCAGAAC
CGCCAACCAGCAAGTGACAGACGCCCTGAACACAAGCCCTCCACACCTGATGGATCTGCTGAGACTGCACAGCAGCCCCTGGCAGGTCTAC
GGCTTTCTGAGAGCCTGTCTGTGCAAGGTGGTGTCCGCATCTCTGTGGGGCACCAGACACAACGAGAGAAGATTCTTCAAGAACCTGAAGAA
GTTCATCAGCCTGGGGAAGTACGGCAAGCTGTCCCTGCAAGAACTGATGTGGAAGATGAAGGTCGAGGACTGCCACTGGCTGAGGTCTAGC

CCTGGCAAGGATAGAGTGCCTGCCGCTGAGCACAGGCTGAGAGAGAATCCTGGCCACATTCCTGTTCTGGCTGATGGACACCTACGTGG
TGCAGCTGCTGCGGAGCTTCTTCTACATCACCGAGAGCACCTTCCAGAAGAACCGGCTGTTCTTCTACCGCAAGAGCGTGTGGTCCAAGCTG
CAGAGCATCGGCGTCAGACAGCACCTGGAAAGAGTGCGCCTGAGAGAGCTGAGCCAAGAGGAAGTGCGGCACCACCAGGATACCTGGCTG
GCCATGCCTATCTGCAGACTGAGATTCATCCCCAAGCCTAACGGCCTGAGGCCTATCGTGAACATGAGCTACAGCATGGGCACTAGAGCCCT
GGGCAGAAGAAAGCAGGCCCAGCACTTCACCCAGAGGCTGAAAACCCTGTTCTCCATGCTGAACTACGAGCGGACAAAGCACCCTCATCTG
ATGGGCTCCTCTGTGCTGGGCATGAACGACATCTACAGGACCTGGCGGGCCTTCGTGCTGAGAGTCAGAGCACTGGACCAGACACCTAGAA
TGTACTTCGTGAAGGCCGACGTGACAGGCGCCTACGATGCTATCCCTCAGGGCAAACTGGTGGAAGTGGTGGCCAACATGATCAGGCACAG
CGAGTCCACCTACTGCATCAGACAGTACGCCGTCGTGCGGAGAGACTCTCAGGGACAAGTGCACAAGAGCTTCCGCAGACAAGTGACCACA
CTGAGCGATCTGCAGCCCTACATGGGCCAGTTTCTGAAGCACCTCCAGGACAGCGACGCCAGCGCTCTGAGAAACTCTGTGGTCATCGAGC
AGTCCATCTCCATGAACGAGTCCAGCTCCAGCCTGTTCGACTTCTTCCTGCACTTCCTGCGGCACAGCGTGGTCAAGATCGGCGACAGATGT
TACACCCAGTGCCAGGGCATCCCACAGGGAAGCAGTCTGAGCACACTGCTGTGCTCCCTGTGTTTCGGCGACATGGAAAACAAGCTGTTCG
CCGAAGTGCAGCGCGACGGACTGCTCCTGAGATTTGTGGACGACTTCCTGCTCGTGACCCCTCACCTGGATCAGGCTAAGACCTTCCTGAG
CACCCTGGTGCATGGCGTGCCAGAGTACGGCTGCATGATCAACCTGCAGAAAACCGTGGTCAACTTCCCCGTGGAACCTGGCACTCTTGGA
GGCGCTGCTCCTTATCAGCTGCCCGCTCATTGTCTGTTCCCTTGGTGCGGACTGCTGCTGGACACACAGACCCTGGAAGTGTTCTGCGACTA
CAGCGGCTACGCCCAGACCAGCATCAAGACCAGCCTGACCTTCCAGTCCGTGTTCAAGGCCGGCAAGACCATGAGGAACAAGCTGCTGAGT
GTGCTGAGGCTGAAGTGCCACGGCCTGTTTCTGGACCTGCAAGTGAACAGCCTGCAGACCGTGTGCATCAATATCTACAAGATCTTTCTGCT
GCAAGCCTACCGGTTCCACGCCTGTGTGATTCAGCTGCCTTTCGACCAGAGAGTGCGGAAGAATCTGACATTCTTTCTGGGCATCATCAGCA
GCCAGGCCAGCTGCTGTTACGCCATCCTGAAAGTGAAGAACCCCGGCATGACCCTGAAGGCCTCTGGCAGCTTTCCACCTGAAGCTGCCCA
TTGGCTGTGCTACCAGGCTTTCCTGCTGAAACTGGCTGCCCACAGCGTGATCTACAAGTGCCTGCTGGGCCCACTGAGAACAGCCCAGAAAC
TGCTGTGTCGGAAGCTGCCTGAAGCCACAATGACAATCCTGAAGGCTGCCGCCGATCCAGCTCTGAGCACCGACTTCCAGACCATCCTGGA
CTGAGAATTCGGATCCGATTCGAGCATTTAAATACGTGGAGCTCGCTGATCAGCCTCGACTGTGCCTTCTAGTTGCCAGCCATCTGTTGTTTG
CCCCTCCCCCGTGCCTTCCTTGACCCTGGAAGGTGCCACTCCCACTGTCCTTTCCTAATAAAATGAGGAAATTGCATCGCATTGTCTGAGTAG
GTGTCATTCTATTCTGGGGGGTGGGGTGGGGCAGGACAGCAAGGGGGAGGATTGGGAAGACAATAGCAGGCATGCTGGGGATGCGGTGGG
CTCTATGGCCACGTGGATATCGCGGCCGCAGGAACCCCTAGTGATGGAGTTGGCCACTCCCTCTCTGCGCGCTCGCTCGCTCACTGAGGCC
GGGCGACCAAAGGTCGCCCGACGCCCGGGCTTTGCCCGGGCGGCCTCAGTGAGCGAGCGAGCGCGCAGCTGCCTGCAGG

SEQ ID NO: 22 (AAV9-CMVenh-MLC2v-moTERT genome)

CCTGCAGGCAGCTGCGCGCTCGCTCGCTCACTGAGGCCGCCCGGGCAAAGCCCGGGCGTCGGGCGACCTTTGGTCGCCCGGCCTCAGTG
AGCGAGCGAGCGCGCAGAGAGGGAGTGGCCAACTCCATCACTAGGGGTTCCTGCGGCCGCGATATCGACATTGATTATTGACTAGTTATTAA
TAGTAATCAATTACGGGGTCATTAGTTCATAGCCCATATATGGAGTTCCGCGTTACATAACTTACGGTAAATGGCCCGCCTGGCTGACCGCCC
AACGACCCCCGCCCATTGACGTCAATAATGACGTATGTTCCCATAGTAACGCCAATAGGGACTTTCCATTGACGTCAATGGGTGGACTATTTA
CGGTAAACTGCCCACTTGGCAGTACATCAAGTGTATCATATGCCAAGTACGCCCCCTATTGACGTCAATGACGGTAAATGGCCCGCCTGGCA
TTATGCCCAGTACATGACCTTATGGGACTTTCCTACTTGGCAGTACATCTACGTATTAGTCATCGCTATTACCATGGTTAACGTTTAAACTTAGA
CAATGGCAGGACCCAGAGCACAGAGCATCGTTCCCAGGCCAGGCCCCAGCCACTGTCTCTTTAACCTTGAAGGCATTTTTGGGTCTCACGTG
TCCACCCAGGCGGGTGTCGGACTTTGAACGGCTCTTACTTCAGAAGAACGGCATGGGGTGGGGGGGCTTAGGTGGCCTCTGCCTCACCTAC
AACTGCCAAAAGTGGTCATGGGGTTATTTTTAACCCCAGGGAAGAGGTATTTATTGTTCCACAGCAGGGGCCGGCCAGCAGGCTCCTTGAAT
TCATTTAAATTCTAGATCGCTAGCACGCGTGCCACCATGACAAGAGCCCCTAGATGTCCTGCCGTCAGAAGCCTGCTGAGAAGCAGATACAG
AGAAGTGTGGCCCCTGGCCACCTTCGTCAGAAGGCTTGGACCTGAAGGCAGACGGCTGGTTCAACCTGGCGACCCCAAGATCTACAGAACC
CTGGTGGCTCAGTGCCTCGTGTGTATGCACTGGGGATCTCAGCCACCTCCTGCCGATCTGAGCTTTCACCAGGTGTCCAGCCTGAAAGAACT
GGTGGCCAGAGTGGTGCAGAGACTGTGCGAGAGAAACGAGAGGAACGTGCTGGCCTTCGGCTTCGAGCTGCTGAATGAAGCTAGAGGCGG
CCCTCCTATGGCCTTCACATCTAGCGTGCGGAGCTACCTGCCTAACACCGTGATCGAGACACTGAGAGTGTCCGGCGCTTGGATGCTGCTGC
TGAGCAGAGTGGGAGATGACCTGCTGGTGTACCTGCTGGCTCACTGTGCCCTCTATCTGCTGGTGCCTCCTAGCTGCGCTTACCAAGTGTGC
GGAAGCCCTCTGTACCAGATCTGTGCCACCACCGACATCTGGCCTAGCGTGTCCGCTTCCTACAGACCCACAAGACCCGTGGGCAGAAACTT
TACCAACCTGAGATTCCTGCAGCAGATCAAGAGCAGCTCCAGGCAAGAGGCCCCTAAGCCTCTTGCTCTGCCTAGCAGAGGCACCAAGAGA
CACCTGAGCCTGACCAGCACAAGCGTGCCCTCTGCCAAGAAAGCCAGATGCTACCCCGTGCCTAGAGTGGAAGAGGGCCCACATAGACAGG
TGCTGCCTACACCTAGCGGCAAGAGCTGGGTTCCATCTCCTGCTAGAAGCCCTGAGGTGCCAACAGCCGAGAAGGACCTGAGCAGCAAGGG
CAAAGTGTCCGACCTGTCTCTGAGCGGCAGCGTGTGTTGCAAGCACAAGCCTAGCAGCACCAGCCTGCTGTCCCCACCTAGACAGAACGCT
TTCCAGCTGAGGCCCTTTATCGAGACAAGACACTTCCTGTACAGCAGAGGCGACGGCCAAGAGAGACTGAACCCTAGCTTCCTGCTGTCTAA

CCTGCAGCCTAACCTGACCGGCGCTAGAAGGCTGGTGGAAATCATCTTCCTGGGCAGCAGACCCAGAACCAGCGGACCTCTGTGTAGAACC
CACAGACTGAGCAGACGGTACTGGCAGATGAGGCCCCTGTTCCAGCAGCTCCTGGTCAACCATGCCGAGTGTCAGTATGTGCGGCTGCTGA
GGTCCCACTGCAGATTCAGAACCGCCAACCAGCAAGTGACAGACGCCCTGAACACAAGCCCTCCACACCTGATGGATCTGCTGAGACTGCA
CAGCAGCCCCTGGCAGGTCTACGGCTTTCTGAGAGCCTGTCTGTGCAAGGTGGTGTCCGCATCTCTGTGGGGCACCAGACACAACGAGAGA
AGATTCTTCAAGAACCTGAAGAAGTTCATCAGCCTGGGGAAGTACGGCAAGCTGTCCCTGCAAGAACTGATGTGGAAGATGAAGGTCGAGGA
CTGCCACTGGCTGAGGTCTAGCCCTGGCAAGGATAGAGTGCCTGCCGCTGAGCACAGGCTGAGAGAGAGAATCCTGGCCACATTCCTGTTC
TGGCTGATGGACACCTACGTGGTGCAGCTGCTGCGGAGCTTCTTCTACATCACCGAGAGCACCTTCCAGAAGAACCGGCTGTTCTTCTACCG
CAAGAGCGTGTGGTCCAAGCTGCAGAGCATCGGCGTCAGACAGCACCTGGAAAGAGTGCGCCTGAGAGAGCTGAGCCAAGAGGAAGTGCG
GCACCACCAGGATACCTGGCTGGCCATGCCTATCTGCAGACTGAGATTCATCCCCAAGCCTAACGGCCTGAGGCCTATCGTGAACATGAGCT
ACAGCATGGGCACTAGAGCCCTGGGCAGAAGAAAGCAGGCCCAGCACTTCACCCAGAGGCTGAAAACCCTGTTCTCCATGCTGAACTACGA
GCGGACAAAGCACCCTCATCTGATGGGCTCCTCTGTGCTGGGCATGAACGACATCTACAGGACCTGGCGGGCCTTCGTGCTGAGAGTCAGA
GCACTGGACCAGACACCTAGAATGTACTTCGTGAAGGCCGACGTGACAGGCGCCTACGATGCTATCCCTCAGGGCAAACTGGTGGAAGTGG
TGGCCAACATGATCAGGCACAGCGAGTCCACCTACTGCATCAGACAGTACGCCGTCGTGCGGAGAGACTCTCAGGGACAAGTGCACAAGAG
CTTCCGCAGACAAGTGACCACACTGAGCGATCTGCAGCCCTACATGGGCCAGTTTCTGAAGCACCTCCAGGACAGCGACGCCAGCGCTCTG
AGAAACTCTGTGGTCATCGAGCAGTCCATCTCCATGAACGAGTCCAGCTCCAGCCTGTTCGACTTCTTCCTGCACTTCCTGCGGCACAGCGT
GGTCAAGATCGGCGACAGATGTTACACCCAGTGCCAGGGCATCCCACAGGGAAGCAGTCTGAGCACACTGCTGTGCTCCCTGTGTTTCGGC
GACATGGAAAACAAGCTGTTCGCCGAAGTGCAGCGCGACGGACTGCTCCTGAGATTTGTGGACGACTTCCTGCTCGTGACCCCTCACCTGG
ATCAGGCTAAGACCTTCCTGAGCACCCTGGTGCATGGCGTGCCAGAGTACGGCTGCATGATCAACCTGCAGAAAACCGTGGTCAACTTCCCC
GTGGAACCTGGCACTCTTGGAGGCGCTGCTCCTTATCAGCTGCCCGCTCATTGTCTGTTCCCTTGGTGCGGACTGCTGCTGGACACACAGAC
CCTGGAAGTGTTCTGCGACTACAGCGGCTACGCCCAGACCAGCATCAAGACCAGCCTGACCTTCCAGTCCGTGTTCAAGGCCGGCAAGACC
ATGAGGAACAAGCTGCTGAGTGTGCTGAGGCTGAAGTGCCACGGCCTGTTTCTGGACCTGCAAGTGAACAGCCTGCAGACCGTGTGCATCA
ATATCTACAAGATCTTTCTGCTGCAAGCCTACCGGTTCCACGCCTGTGTGATTCAGCTGCCTTTCGACCAGAGAGTGCGGAAGAATCTGACAT
TCTTTCTGGGCATCATCAGCAGCCAGGCCAGCTGCTGTTACGCCATCCTGAAAGTGAAGAACCCCGGCATGACCCTGAAGGCCTCTGGCAG
CTTTCCACCTGAAGCTGCCCATTGGCTGTGCTACCAGGCTTTCCTGCTGAAACTGGCTGCCCACAGCGTGATCTACAAGTGCCTGCTGGGCC
CACTGAGAACAGCCCAGAAACTGCTGTGTCGGAAGCTGCCTGAAGCCACAATGACAATCCTGAAGGCTGCCGCCGATCCAGCTCTGAGCAC
CGACTTCCAGACCATCCTGGACTGAGAATTCGGATCCGATTCGAGCATTTAAATACGTGGAGCTCGCTGATCAGCCTCGACTGTGCCTTCTAG
TTGCCAGCCATCTGTTGTTTGCCCCTCCCCCGTGCCTTCCTTGACCCTGGAAGGTGCCACTCCCACTGTCCTTTCCTAATAAAATGAGGAAAT
TGCATCGCATTGTCTGAGTAGGTGTCATTCTATTCTGGGGGGTGGGGTGGGGCAGGACAGCAAGGGGGAGGATTGGGAAGACAATAGCAGG
CATGCTGGGGATGCGGTGGGCTCTATGGCCACGTGGATATCGCGGCCGCAGGAACCCCTAGTGATGGAGTTGGCCACTCCCTCTCTGCGC
GCTCGCTCGCTCACTGAGGCCGGGCGACCAAAGGTCGCCCGACGCCCGGGCTTTGCCCGGGCGGCCTCAGTGAGCGAGCGAGCGCGCAG
CTGCCTGCAGG

SEQ ID NO: 23 (AAV9-TNT-moTert genome)

CCTGCAGGCAGCTGCGCGCTCGCTCGCTCACTGAGGCCGCCCGGGCAAAGCCCGGGCGTCGGGCGACCTTTGGTCGCCCGGCCTCAGTG

AGCGAGCGAGCGCGCAGAGAGGGAGTGGCCAACTCCATCACTAGGGGTTCCTGCGGCCGCGATATCCTCAGTCCATTAGGAGCCAGTAGC

CTGGAAGATGTCTTTACCCCCAGCATCAGTTCAAGTGGAGCAGCACATAACTCTTGCCCTCTGCCTTCCAAGATTCTGGTGCTGAGACTTATG

GAGTGTCTTGGAGGTTGCCTTCTGCCCCCCAACCCTGCTCCCAGCTGGCCCTCCCAGGCCTGGGTTGCTGGCCTCTGCTTTATCAGGATTCT

CAAGAGGGACAGCTGGTTTATGTTGCATGACTGTTCCCTGCATATCTGCTCTGGTTTTAAATAGCTTATCTGAGCAGCTGGAGGACCACATGG

GCTTATATGGCGTGGGGTACATGTTCCTGTAGCCTTGTCCCTGGCACCTGCCAAAATAGCAGCCAACACCCCCCACCCCCACCGCCATCCCC

CTGCCCCACCCGTCCCCTGTCGCACATTCCTCCCTCCGCAGGGCTGGCTCACCAGGCCCCAGCCCACATGCCTGCTTAAAGCCCTCTCCAT

CCTCTGCCTCACCCAGTCCCCGCTGAGACTGAGCAGACGCCTCCAGGATCTGTCGGCAGGAATTATCGCTAGCACGCGTGCCACCATGACA

AGAGCCCCTAGATGTCCTGCCGTCAGAAGCCTGCTGAGAAGCAGATACAGAGAAGTGTGGCCCCTGGCCACCTTCGTCAGAAGGCTTGGAC

CTGAAGGCAGACGGCTGGTTCAACCTGGCGACCCCAAGATCTACAGAACCCTGGTGGCTCAGTGCCTCGTGTGTATGCACTGGGGATCTCA

GCCACCTCCTGCCGATCTGAGCTTTCACCAGGTGTCCAGCCTGAAAGAACTGGTGGCCAGAGTGGTGCAGAGACTGTGCGAGAGAAACGAG

AGGAACGTGCTGGCCTTCGGCTTCGAGCTGCTGAATGAAGCTAGAGGCGGCCCTCCTATGGCCTTCACATCTAGCGTGCGGAGCTACCTGC

CTAACACCGTGATCGAGACACTGAGAGTGTCCGGCGCTTGGATGCTGCTGCTGAGCAGAGTGGGAGATGACCTGCTGGTGTACCTGCTGGC

TCACTGTGCCCTCTATCTGCTGGTGCCTCCTAGCTGCGCTTACCAAGTGTGCGGAAGCCCTCTGTACCAGATCTGTGCCACCACCGACATCT

GGCCTAGCGTGTCCGCTTCCTACAGACCCACAAGACCCGTGGGCAGAAACTTTACCAACCTGAGATTCCTGCAGCAGATCAAGAGCAGCTCC

AGGCAAGAGGCCCCTAAGCCTCTTGCTCTGCCTAGCAGAGGCACCAAGAGACACCTGAGCCTGACCAGCACAAGCGTGCCCTCTGCCAAGA
AAGCCAGATGCTACCCCGTGCCTAGAGTGGAAGAGGGCCCACATAGACAGGTGCTGCCTACACCTAGCGGCAAGAGCTGGGTTCCATCTCC
TGCTAGAAGCCCTGAGGTGCCAACAGCCGAGAAGGACCTGAGCAGCAAGGGCAAAGTGTCCGACCTGTCTCTGAGCGGCAGCGTGTGTTGC
AAGCACAAGCCTAGCAGCACCAGCCTGCTGTCCCCACCTAGACAGAACGCTTTCCAGCTGAGGCCCTTTATCGAGACAAGACACTTCCTGTA
CAGCAGAGGCGACGGCCAAGAGAGACTGAACCCTAGCTTCCTGCTGTCTAACCTGCAGCCTAACCTGACCGGCGCTAGAAGGCTGGTGGAA
ATCATCTTCCTGGGCAGCAGACCCAGAACCAGCGGACCTCTGTGTAGAACCCACAGACTGAGCAGACGGTACTGGCAGATGAGGCCCCTGT
TCCAGCAGCTCCTGGTCAACCATGCCGAGTGTCAGTATGTGCGGCTGCTGAGGTCCCACTGCAGATTCAGAACCGCCAACCAGCAAGTGAC
AGACGCCCTGAACACAAGCCCTCCACACCTGATGGATCTGCTGAGACTGCACAGCAGCCCCTGGCAGGTCTACGGCTTTCTGAGAGCCTGT
CTGTGCAAGGTGGTGTCCGCATCTCTGTGGGGCACCAGACACAACGAGAGAAGATTCTTCAAGAACCTGAAGAAGTTCATCAGCCTGGGGAA
GTACGGCAAGCTGTCCCTGCAAGAACTGATGTGGAAGATGAAGGTCGAGGACTGCCACTGGCTGAGGTCTAGCCCTGGCAAGGATAGAGTG
CCTGCCGCTGAGCACAGGCTGAGAGAGAGAATCCTGGCCACATTCCTGTTCTGGCTGATGGACACCTACGTGGTGCAGCTGCTGCGGAGCT
TCTTCTACATCACCGAGAGCACCTTCCAGAAGAACCGGCTGTTCTTCTACCGCAAGAGCGTGTGGTCCAAGCTGCAGAGCATCGGCGTCAGA
CAGCACCTGGAAAGAGTGCGCCTGAGAGAGCTGAGCCAAGAGGAAGTGCGGCACCACCAGGATACCTGGCTGGCCATGCCTATCTGCAGA
CTGAGATTCATCCCCAAGCCTAACGGCCTGAGGCCTATCGTGAACATGAGCTACAGCATGGGCACTAGAGCCCTGGGCAGAAGAAAGCAGG
CCCAGCACTTCACCCAGAGGCTGAAAACCCTGTTCTCCATGCTGAACTACGAGCGGACAAAGCACCCTCATCTGATGGGCTCCTCTGTGCTG
GGCATGAACGACATCTACAGGACCTGGCGGGCCTTCGTGCTGAGAGTCAGAGCACTGGACCAGACACCTAGAATGTACTTCGTGAAGGCCG
ACGTGACAGGCGCCTACGATGCTATCCCTCAGGGCAAACTGGTGGAAGTGGTGGCCAACATGATCAGGCACAGCGAGTCCACCTACTGCAT
CAGACAGTACGCCGTCGTGCGGAGAGACTCTCAGGGACAAGTGCACAAGAGCTTCCGCAGACAAGTGACCACACTGAGCGATCTGCAGCCC
TACATGGGCCAGTTTCTGAAGCACCTCCAGGACAGCGACGCCAGCGCTCTGAGAAACTCTGTGGTCATCGAGCAGTCCATCTCCATGAACGA
GTCCAGCTCCAGCCTGTTCGACTTCTTCCTGCACTTCCTGCGGCACAGCGTGGTCAAGATCGGCGACAGATGTTACACCCAGTGCCAGGGCA
TCCCACAGGGAAGCAGTCTGAGCACACTGCTGTGCTCCCTGTGTTTCGGCGACATGGAAAACAAGCTGTTCGCCGAAGTGCAGCGCGACGG
ACTGCTCCTGAGATTTGTGGACGACTTCCTGCTCGTGACCCCTCACCTGGATCAGGCTAAGACCTTCCTGAGCACCCTGGTGCATGGCGTGC
CAGAGTACGGCTGCATGATCAACCTGCAGAAAACCGTGGTCAACTTCCCCGTGGAACCTGGCACTCTTGGAGGCGCTGCTCCTTATCAGCTG
CCCGCTCATTGTCTGTTCCCTTGGTGCGGACTGCTGCTGGACACACAGACCCTGGAAGTGTTCTGCGACTACAGCGGCTACGCCCAGACCA
GCATCAAGACCAGCCTGACCTTCCAGTCCGTGTTCAAGGCCGGCAAGACCATGAGGAACAAGCTGCTGAGTGTGCTGAGGCTGAAGTGCCA
CGGCCTGTTTCTGGACCTGCAAGTGAACAGCCTGCAGACCGTGTGCATCAATATCTACAAGATCTTTCTGCTGCAAGCCTACCGGTTCCACG
CCTGTGTGATTCAGCTGCCTTTCGACCAGAGAGTGCGGAAGAATCTGACATTCTTTCTGGGCATCATCAGCAGCCAGGCCAGCTGCTGTTAC
GCCATCCTGAAAGTGAAGAACCCCGGCATGACCCTGAAGGCCTCTGGCAGCTTTCCACCTGAAGCTGCCCATTGGCTGTGCTACCAGGCTTT
CCTGCTGAAACTGGCTGCCCACAGCGTGATCTACAAGTGCCTGCTGGGCCCACTGAGAACAGCCCAGAAACTGCTGTGTCGGAAGCTGCCT
GAAGCCACAATGACAATCCTGAAGGCTGCCGCCGATCCAGCTCTGAGCACCGACTTCCAGACCATCCTGGACTGAGAATTCGGATCCGATTC
GAGCATTTAAATACGTGGAGCTCGCTGATCAGCCTCGACTGTGCCTTCTAGTTGCCAGCCATCTGTTGTTTGCCCCTCCCCCGTGCCTTCCTT
GACCCTGGAAGGTGCCACTCCCACTGTCCTTTCCTAATAAAATGAGGAAATTGCATCGCATTGTCTGAGTAGGTGTCATTCTATTCTGGGGGG
TGGGGTGGGGCAGGACAGCAAGGGGGAGGATTGGGAAGACAATAGCAGGCATGCTGGGGATGCGGTGGGCTCTATGGCCACGTGGATATC
GCGGCCGCAGGAACCCCTAGTGATGGAGTTGGCCACTCCCTCTCTGCGCGCTCGCTCGCTCACTGAGGCCGGGCGACCAAAGGTCGCCCG
ACGCCCGGGCTTTGCCCGGGCGGCCTCAGTGAGCGAGCGAGCGCGCAGCTGCCTGCAGG

## Claims

1. A recombinant viral genome comprising a nucleotide sequence encoding a telomerase reverse transcriptase (TERT) operably linked to a tissue-specific and/or organ-specific promoter, wherein the total length of the viral genome is less than 4700 nucleotides.

2. A recombinant viral genome according to claim 1, wherein the tissue-specific and/or organ-specific promoter is a lung-specific promoter or a heart-specific promoter.

3. A recombinant viral genome according to claim 1 or 2, wherein said genome is a recombinant adeno-associated virus genome.

4. A recombinant viral genome according to any one of claims 1-3, wherein the promoter is selected from the group consisting of an SpB promoter, a CMVenh-MLC2V promoter, a troponin promoter and derivatives thereof.

5. A recombinant viral genome according to any one of claims 1-4, wherein the TERT encoding sequence is codon-optimized, preferably for expression in a human cell.

6. A recombinant viral genome according to any one of claims 1-5, wherein:
a) the TERT encoding sequence comprises, essentially consists of, or consists of a nucleotide sequence encoding a polypeptide represented by an amino acid sequence having at least 60%, 70%, 80%, 90%, 95%, or 99% identity or similarity with any one of SEQ ID NOs: 4, 7, or 10, preferably with SEQ ID NO: 7 or 10, or;
b) the TERT encoding sequence comprises, essentially consists of, or consists of a nucleotide sequence having at least 60%, 70%, 80%, 90%, 95%, or 99% identity with any one of SEQ ID NOs: 1, 2, 3, 5, 6, 8, or 9, preferably with any one of SEQ ID NOs: 3, 6, or 9, or;
c) the TERT encoding sequence comprises, essentially consists of, or consists of a nucleotide sequence the sequence of which differs from the sequence of a nucleotide sequence of a) or b) due to the degeneracy of the genetic code.

7. A recombinant viral genome according to any one of claims 1-6, wherein the promoter comprises, essentially consists of, or consists of a nucleotide sequence having at least 60%, 70%, 80%, 90%, 95%, or 99% identity with anyone of the nucleotide sequences with SEQ ID NOs: 11, 12, 13, 15, or 16, preferably with SEQ ID NOs: 12, 15, or 16.

8. A recombinant viral genome according to any one of claims 1-7, wherein the recombinant viral genome further comprises a kozak consensus sequence operably linked to the nucleotide sequence encoding a TERT.

9. A recombinant viral genome according to any one of claims 1-8, wherein the recombinant viral genome further comprises a polyA sequence operably linked to the nucleotide sequence encoding a TERT.

10. An adeno-associated viral vector comprising a recombinant viral genome as defined in any one of claims 1-9.

11. An adeno-associated viral vector according to claim 10, wherein the viral vector is of serotype 1, 2, 3, 4, 5, 6, 7, 8, 9, rh10, rh8, Cb4, rh74, DJ, 2/5, 2/1, 1/2 or Anc80, preferably of serotype 6 or 9.

12. A pharmaceutical composition comprising a recombinant viral genome according to any one of claims 1-9 or an adeno-associated viral vector according to claims 10-11, optionally further comprising one or more pharmaceutically acceptable ingredients.

13. A recombinant viral genome according to any one of claims 1-9, an adeno-associated viral vector according to any one of claims 10-11, or a pharmaceutical composition according to claim 12, for use as a medicament.

14. A recombinant viral genome according to any one of claims 1-9, an adeno-associated viral vector according to claims 10-11, or a pharmaceutical composition according to claim 12, for use in the treatment and/or prevention of a condition associated with shortened telomere length.

15. A recombinant viral genome according to any one of claims 1-9, an adeno-associated viral vector according to claims 10-11, or a pharmaceutical composition according to claim 12, for use in the treatment and/or prevention of pulmonary fibrosis, myocardial infarction or conditions associated therewith.
